# EUROPEAN PATENT APPLICATION

(11) **EP 1 336 655 A1**
(43) Date of publication of application: **20.08.2003**
(21) Application number: 02000733.2
(22) Date of filing: 12.01.2002
(51) Int. Cl.: C12N 15/10, G01N 33/76

(54) **Method of identifying protein CAMS (constitutively active mutants)**

(71) Applicant: Aventis Pharma Deutschland GmbH, 65929 Frankfurt am Main (DE)
(72) Inventor: Fraissignes, Pauline, 13006 Marseille (FR); Gratzer, Sabine, 82166 Gräfelfing (DE); Leberer, Ekkehard, 82100 Germering (DE)

(57) **Abstract**

The invention relates to a method of identifying protein CAMs (constitutively active mutants) wherein a library of mutated sequences of a protein is generated, yeast cells are transformed with such a library, and the respective protein CAM is identified. Moreover the invention relates to the use of such a method for identifying agonists or inverse agonists.

## Description

The present invention relates to a method of identifying protein CAMs and the use thereof.

The G protein-coupled receptors (GPCRs) constitute the largest family of membrane receptors with a common evolutionary origin. They include receptors which respond to environmental ligands (odorants, flavors) or radiations (light of various wavelengths) and to innumerable internal signals (hormones, bioactive amines, neuropeptides, arachidonic acid metabolites, purines, etc...). This extreme diversity contrasts with their stereotyped structure (seven transmembrane alpha helices, three extracellular loops, three intracellular loops, amino terminus outside and carboxyl terminus inside the cell) and with the limited number of downstream regulatory cascades they control.
The GPCR interacts with an intracellular heterotrimeric G protein consisting of αβγ subunits. Upon binding of the receptor's ligand, the α-subunit dissociates from the β-and γ-subunits, and hydrolyses GTP to GDP. Both Gα and Cβγ can then activate downstream transduction effectors or regulate other receptors (Zwick *et al*., 1999). In haploid *Saccharomyces cerevisiae* cells, GPCRs are regulating the mating process. The receptor (Ste2 or Ste3) detects the presence of cells of the opposite mating type (through binding of peptide mating pheromones), and activates intracellular heterotrimeric G proteins, thus initiating the mating process. Gpa1 (α subunit) dissociates from the βγ (Ste4-Ste18) complex which activates downstream elements of the pheromone response pathway which includes a well-characterized mitogen-activated protein kinase (MAP kinase) cascade. The transcription factor Ste12 can then initiate the transcription of several mating factor-inducible genes such as *FUS1*.

Reports of mammalian GPCRs expressed in yeast indicate that these heterologous proteins can be reliably expressed in yeast and properly inserted into yeast membranes (Tate and Grisshammer, 1996). Reports, e.g. (Price *et al*., 1995), demonstrate that a large number of heterologous GPCRs interact with the yeast heterotrimeric G protein with sufficient efficacy to induce a growth-promoting signal. In case the GPCR under investigation does not couple to Gpa1, it is co-expressed together with a chimera where the C-terminal part of Gpa1 is replaced by the corresponding amino acids of a given human G_{α} subunit (Brown *et al.,* 2000). A reporter construct (such as *pFUS1-HIS3* or *pFUS1-lacZ)* is then expected to produce a detectable response upon receptor activation.

Increasingly, it is being appreciated that endogenous receptors, and in particular those of the G protein-coupled receptor family, may possess some level of constitutive activity even in the absence of activating mutation. A potentially important physiological ramification of the constitutive activity of such receptors is that the ability of different receptor subtypes (for the same ligand) to spontaneously isomerize to the active state might well differ. Such receptor subtypes would vary substantially in their properties, thus best suiting them for one or another physiological context (Lefkowitz *et al*., 1993).

The discovery of constitutive GPCR activity presents a theoretical approach to the identification of ligands for orphan receptors. The basic premise for this idea is that different tertiary conformations (i.e. different allosteric change states) of the receptor protein will display different binding domains for ligands, or different binding affinities for the same ligand. Since the mutation of a receptor sequence can only affect the physico-chemical properties of the receptor, but not those of ligands, a change of affinity of a ligand for a receptor ought to be of a similar magnitude for all ligands and not proportional to the ligand's efficacy (Lefkowitz *et al.,* 1993).

The notion that constitutive activation of G protein-coupled receptors could be responsible for hereditary diseases came first from the study of patients suffering of retinis pigmentosa (Robinson *et al*., 1992). Since then, several other human pathologies have been linked to constitutive activity or aberrant receptors (Dhanasekaran *et al.,* 1995) (Rao and Oprian, 1996) (Duprez *et al*., 1997) (Jensen et *al*., 2000). It has now been recognized that very valuable information relevant to treatment of diseases caused by constitutively active receptors (for instance TSH and LH receptors (Spiegel, 1996)) can be directly obtained by identifying compounds which act as inverse agonists to constitutively activated forms of the receptor.

Additionally, some of the therapeutic effects of presently used receptor antagonists may be related to their inverse agonist properties. Recent results (Varma *et al.,* 1999) show that almost all β-adrenergic antagonists (with the exception of pindolol) have inverse agonist properties in the heart of reserpine treated rats.

Down regulation and desensitization of GPCRs is elicited by agonists or as a consequence of spontaneous activity. Thus, inverse agonists upregulate heptahelical receptors by decreasing spontaneous downregulation (Daeffler and Landry, 2000), offering new approaches to tolerance and dependence to drugs.
Amino acid residues can be mutated and lead to ligand-independent activation of the receptor and constitutive activation of signaling pathway (Lefkowitz *et al*., 1993) (Rao and Oprian, 1996) (Sommers *et al*., 2000) (Konopka *et al*., 1996) (Alewijnse *et al*., 2000).

Several techniques have been applied to the discovery or the study of constitutively active receptors. For instance, manipulation of the stoichiometry of receptors and G proteins (mainly over-expression of the receptor) can create a constitutive active receptor system (Chen *et al*., 2000) (Samama *et al.,* 1997) or site directed mutagenesis on residues such as the highly conserved DRY motif were found to be involved in stabilizing intramolecular interactions (Alewijnse *et al*., 2000).
To date, random mutagenesis has been used in many works to identify CAMs : in a random saturation mutagenesis of a critical region of the Calcium-sensing receptor (Jensen *et al.,* 2000) or in a systematic screening in a mammalian cell based bioassay of a random mutant library of the angiotensin II AT_{1A} receptor (Parnot *et al.,* 2000).
The ease of genetic manipulation of yeast and the availability of an assay that allows detection of a signaling activity made it possible to search through large random mutational libraries to study the spectrum of mutations capable of causing constitutive activation. Ma & al. (Ma *et al.,* 1987) described a fast and reliable method for plasmid construction (Gap Repair) that is based on the efficient repair of a linearized plasmid by recombination with a homologous DNA restriction fragment during yeast transformation.
Additionally, *S. cerevisiae* does not show such a rapid desensitization process comparable to the ligand-dependent phosphorylation of receptors followed by receptor interaction with arrestins, disruption of the interaction receptor-G protein, and in some case sequestration (Tsao *et al.,* 2001). This makes the identification of a constitutive activity much easier.

Previously, a random mutagenesis strategy combined with a yeast based *in vivo* sub-cloning/screening has been applied successfully on the amino-terminal and transmembrane regions (approximately the first 300 out of 431 residues) of the yeast Ste2 G protein-coupled receptor (Sommers *et al.,* 2000) and on the second intracellular loop of the V2 Vasopressin receptor (Erlenbach *et al*., 2001). In contrast, the present method allows the systematic identification of activating mutations over the whole open reading frame, without the need of focusing on some regions. Although people usually choose to mutagenise only a part of the coding sequence because they believe that only this region is involved in the mechanism studied (Erlenbach *et al.,* 2001), no doubt would persist and sometimes new prospects on structure-activity would appear.

WO 00/12705 discloses methods for improving the function of heterologous G protein-coupled receptors.

Random mutagenesis on human GPCRs and functionally studied in mammalian cells, were described by (Parnot *et al*., 2000)) (CAM discovery of Angiotensin II 1A receptor, full length) and (Jensen *et al*., 2000) (Functional Importance of the Ala¹¹⁶-Pro¹³⁶ Region in the Calcium-sensing Receptor).

Random mutagenesis of a yeast GPCR and functionally studied in yeast cells, in particular CAM discovery of Ste2 (α-factor receptor), random mutagenesis of amino-terminal and transmembrane regions, including Gap Repair were described by (Sommers *et al.,* 2000) and (Sommers and Dumont, 1997)).

Random mutagenesis on a human GPCR functionally studied in yeast cells, in particular coupling properties study of V2 vasopressin receptor, oligonucleotide-directed random mutagenesis of the intracellular loop 2 (228 bp), including Gap repair were described by (Erlenbach *et al*., 2001)).

CAMs and methods of using them are also disclosed in WO 00/21987. WO 00/06597 discloses endogenous constitutively activated G protein-coupled orphan receptors. WO 00/22129 and WO 00/22131 disclose non-endogenous constitutively activated human G protein-coupled orphan receptors (site directed mutagenesis of GPCRs to generate constitutively activated mutants) and WO 97/21731 an assay for and uses of peptide hormone receptor ligands.

The discovery of constitutively activated mutants (CAMs) is usually the result of a long process of genetic manipulations and assays in mammalian cell culture. Researchers usually choose site directed mutagenesis because of its more straight forward and fast principle (Egan *et al*., 1998;Alewijnse *et al*., 2000).

It was a task of the present invention to provide an easy and fast method for identifying CAMs of proteins, e.g. for GPCRs, ion-channel, enzymes.

The present invention provides a method for identifying protein CAMs (constitutively active mutants), wherein
a) a library of mutated sequences of a protein is generated,
b) yeast cells are transformed with such library and
c) the respective protein CAM is identified.

Examples for proteins for which CAMs can be identified are GPCRs, ion-channels, enzymes, e.g. kinases, proteases, transcription factors.

Preferably, protein CAMs of mammalian proteins are identified, e.g. CAMs of human proteins.

The present invention provides a method of identifying protein CAMs (constitutively active mutants) wherein
a) a library of mutated sequences of a protein is generated,
b) yeast cells are co-transformed with the library and a linearized expression vector,
c) the transformed yeast cells are selected for the repair of the plasmid, and
d) protein CAMs are identified by determining the activity of the respective protein mutant.

The present invention provides a method of identifying protein CAMs (constitutively active mutants) wherein
a) a library of mutated sequences of a protein is generated,
b) yeast cells are co-transformed with such library and a linearized expression vector,
c) the transformed yeast cells are selected for the repair of the plasmid, and
d) the previously selected yeast colonies are transferred on a second selective plate where they are screened for the activity of the respective protein mutant.

In a special embodiment of the invention low fidelity PCR is applied on a full length sequences of a particular protein, e.g. a GPCR, preferably a mammalian protein sequence. The PCR products were co-transformed with a linearized expression vector (e.g. containing at each end short sequences homologous to the end of the PCR product) into an engineered yeast strain. The transformed yeast cells were first selected for the repair of the plasmid (e.g. selection by colony forming on a selective medium). The colonies previously selected were replicated an another medium, selective for the activity of the protein, e.g. the receptor (e.g. by the use of a survival reporter gene expressed only upon receptor signaling). Preferably, three or more identical and independent experiments were done to avoid the PCR's bias. The protein CAMs ("mutants") have an increased basal signaling activity and the same maximum of stimulation than the wild type protein.

A yeast based *in vivo* discovery of random active mutants can be applied to the entire coding sequence of a protein, e.g. a human receptor. This was done by screening for constitutive mutations of the human sphingosine 1-phosphate receptor EDG5 (Endothelial Differentiation Gene 5) (An *et al*., 2000) (Hla, 2001).

To obtain a whole set of single point mutants directly (and also to avoid excessive secondary sub-cloning work to find out the activity conferred by each single point mutation), the PCR protocol was optimized to induce an average of less than one point mutation per copy of the gene. Indeed, the high throughput potential of an *in vivo* subcloning/screening strategy allows us to increase the size of the library without consuming more time/money.

In another embodiment the present invention relates to engineered yeast cells comprising a library of mutants (e.g. GPCR CAMs) and the use of such engineered yeast cells.

For such engineering for example Saccharomyces cerevisiae, Schizosaccharomyces pombe and Candida albicans cells can be used.

The use of such an engineered yeast cell should bring three major improvements at the same time:
- screening of a whole library of mutants generated by low fidelity polymerase chain reaction (PCR)
- in vivo sub-cloning of each mutated sequences into the expression vector by homologous recombination
- in vivo selection of the active mutants using reporter genes All three in the same engineered yeast cell.

Further advantages are, that the yeast is a powerful tool for the study of mammalian GPCRs and their transduction characteristics because of the high homology between these eukaryotic cells (Price *et al.,* 1995;Hadcock and Pausch, 1999;Botstein *et al*., 1997); Yeast has a high rate of homologous recombination and the genetic manipulations of yeast are easy (Ma *et al*., 1987;Oldenburg *et al*., 1997); Yeast allows in vivo selection of a receptor's activity (Chambers *et al.,* 2000); In vivo screen allows the direct recovery of the plasmid carrying the mutant of interest (CAM) from the micro-organism. Yeast is cheaper to cultivate and engineer than mammalian cells. The technology used to sub-clone and detect the mutants' activity in mammalian cells is far more expensive and qualitative selection and quantification of the mutants' activity can both be done in the same yeast system.

The present method of identifying protein CAMs presents a low cost, fast and powerful method to systematically identify activating mutations along the whole coding sequence of a protein. In contrast to previous work (Parnot *et al*., 2000), the cloning step is simplified to a simple transformation in yeast and the selection of active mutants is not more than picking growing colonies.

The transposition of the method into mammalian cells confirmed very well the constitutive activity of the mutants screened and selected in yeast. This proves that the method is a suitable alternative to mutant screening in mammalian systems.

Another big advantage of the method is that it immediately discriminates between a moderately active and a highly active mutant (a too high basal activity would not be suitable for agonist discovery, but appropriate for inverse-agonist screening). The growth speed of the colonies on agar selective medium is well correlated to the different "intensities" of constitutive activity observed in a liquid reporter assay.

De-orphaning can also be achieved with this method, e.g. the method can be applied to orphan GPCRs. Therefore, a low fidelity PCR product was co-transfected with the linearized vector into a panel of yeast strains expressing different humanized Gα protein subunits. On selective medium, mutants were selected only from the yeast strain expressing the Gα specific for its coupling. β-Galactosidase detection after growth in a selective medium showed an increased basal activity of the receptor mutant (i.e. an increased expression level of lacZ, controlled by a FUS1 promoter).

The method of identifying protein CAMs, of e.g. GPCR CAMs can be used for:
- Identifying agonists; such use is based on the fact that the affinity of a protein CAM, e.g. GPCR CAM for his agonist is increased (Lefkowitz *et al*., 1993) (MacEwan and Milligan, 1996) (Alewijnse *et al*., 2000);
- Identifying inverse agonists (Chen *et al*., 2000);
- Studying proteins oligomerization, e.g. GPCR oligomerization, depending on their state of activity;
- Crystallizing protein, e.g. GPCRs in different tertiary conformations;
- De-orphaning: modulators of protein action can be identified with no prior knowledge of the endogenous ligand or protein function.

### Figures:

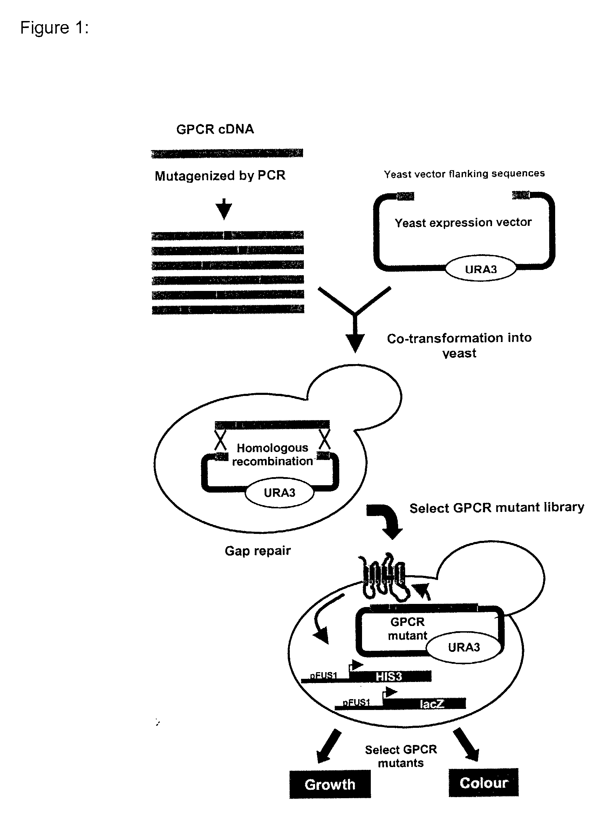

### Figure 1: Summary of the method of identifying GPCR CAMs.

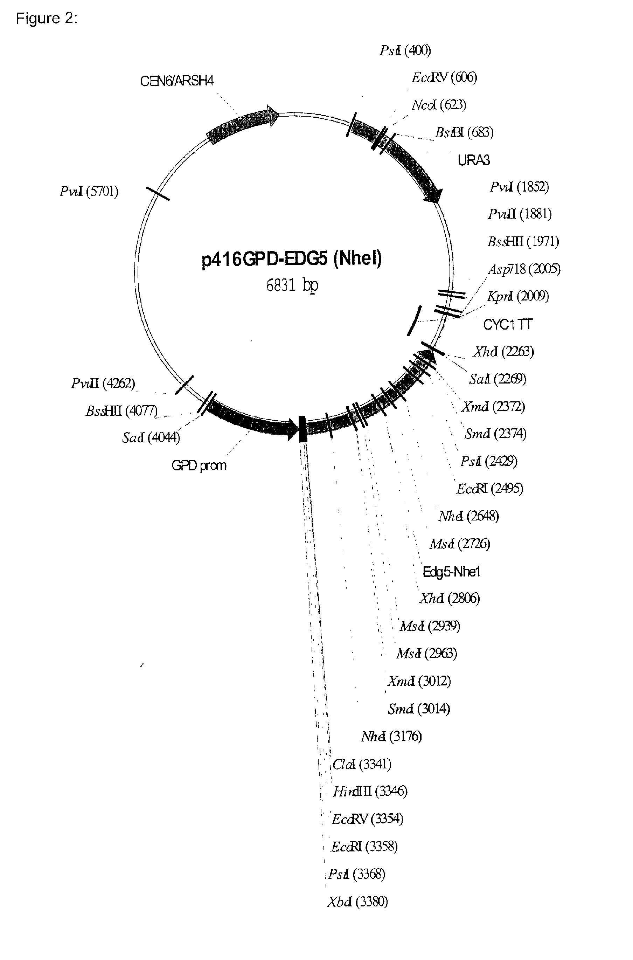

Figure 1 summarizes the whole process of the method.
Random mutagenesis of the *EDG5* gene was conducted using the yeast *CEN* expression plasmid p416GPD-Edg5 carrying an *URA3* marker (Figure 2).

### Figure 2: Restriction map of p416GPD-Edg5 (Nhel)

A Nhel restriction site was created at position 157 bp of the coding sequence of *EDG5.* Three nucleotides where exchanged by site directed mutagenesis to create the site. This was necessary to conserve, after linearization of the plasmid, only the first 157 bp and the last 101 bp of the open reading frame for homologous recombination.

To allow *in vivo* recombination, the p416GPD-Edg5 was linearized by double digestion Nhel-Xmal before co-transformation with the low-fidelity PCR amplification product.

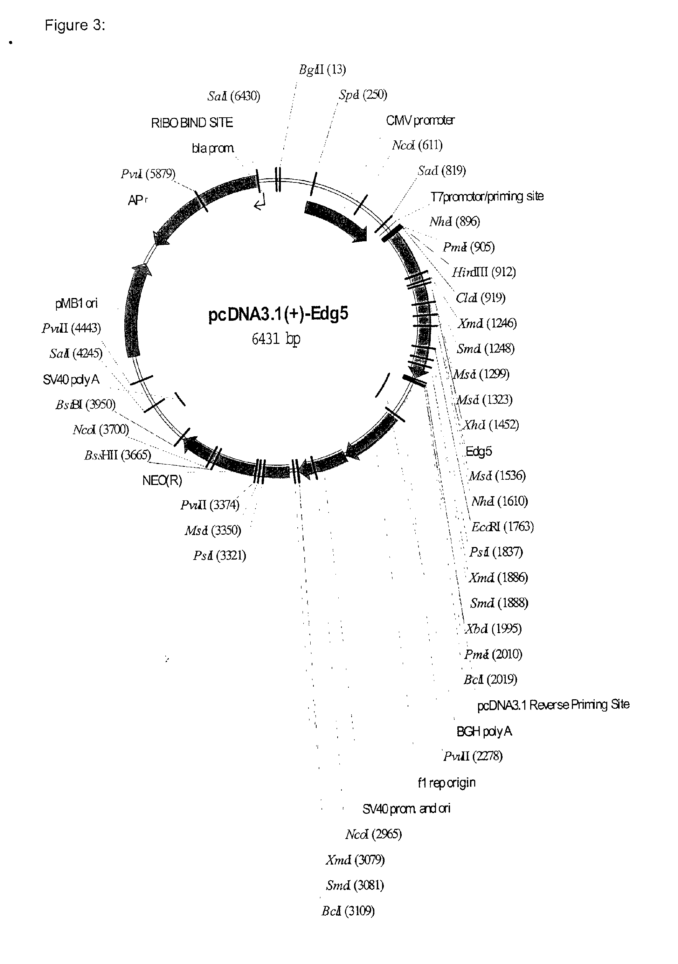

### Figure 3: Restriction map of pcDNA3.1(+)-Edg5

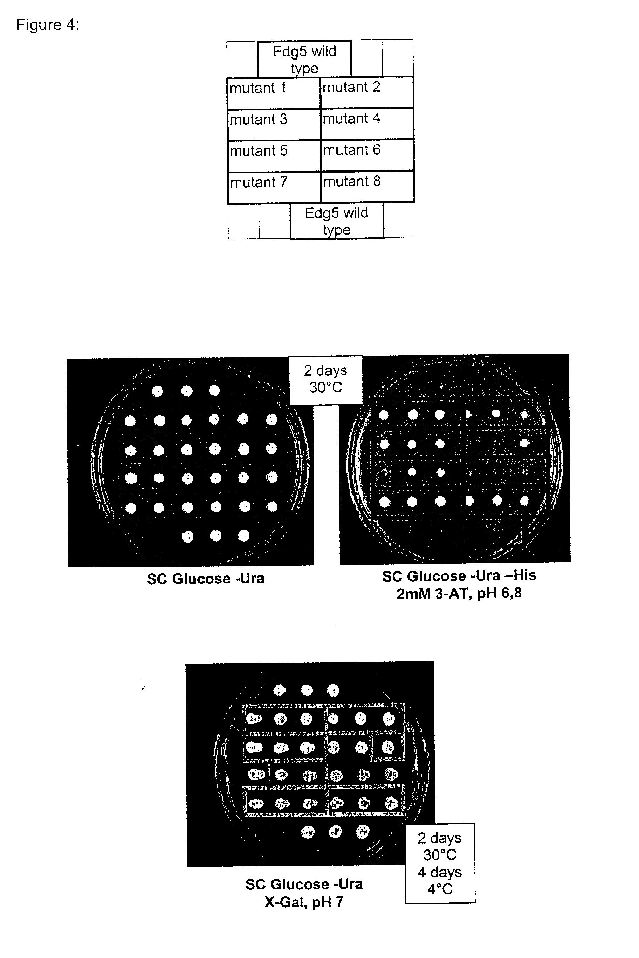

### Figure 4: Solid phase assay

Three colonies of each yeast transformation were tested for the growth and for the β-Galactosidease activity on selective plate:
- the first plate (SC Glucose -Ura) shows the normal growth of the colonies;
- the second plate (SC Glucose -Ura -His containing 2mM 3-AT, pH 6.8) shows the growth of the colonies expressing a constitutively activated mutant;
- the third plate (SC Glucose -Ura, X-Gal, pH 7) is testing the β-Galactosidase activity (its substrate X-Gal is transformed in a blue metabolite) of the mutants:
   the frame shows the colored colonies which correspond to the most active clones
   and correlates with the observations from the second plate.

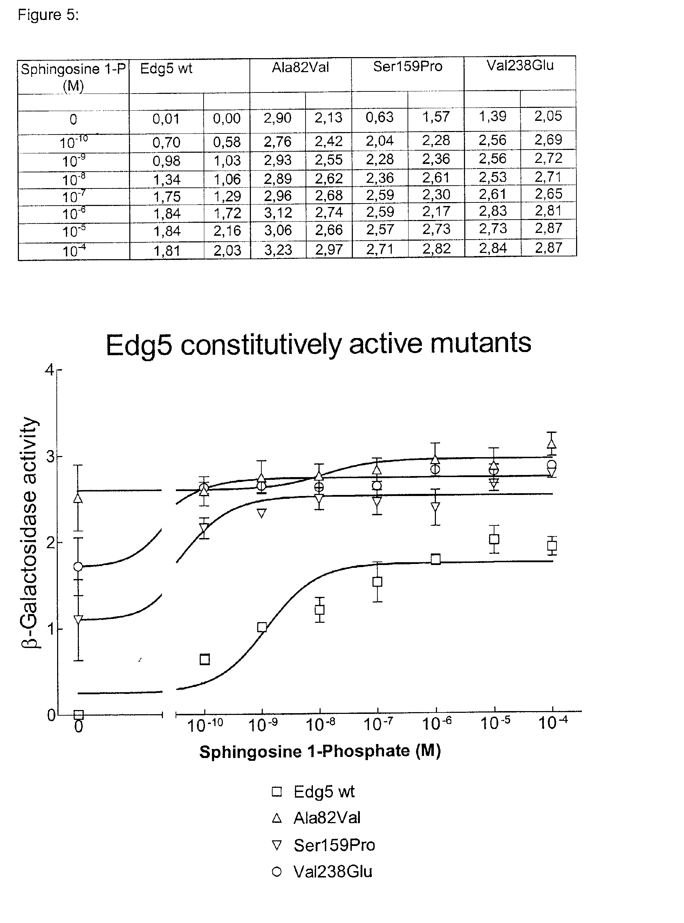

### Figure 5: Liquid assay

After 24 hours of growth of the different mutants in selective liquid medium, in the presence of an increasing concentration of Sphingosine 1-Phosphate, β-Galactosidase activity was measured in a colorimetric assay by adding the substrate CPRG, incubating 2 hours and measuring the absorbance at 574nm.

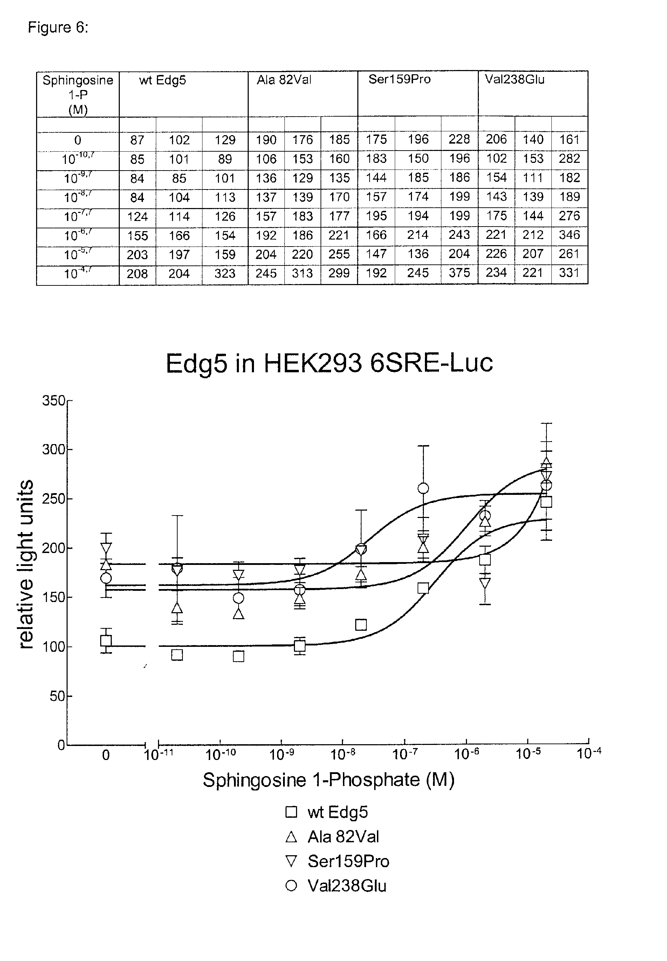

### Figure 6: Cell culture assay

Luciferase activity measured in triplicates after 24 hours of stimulation by a serial dilution of Sphingosine 1-Phosphate.

### Examples:

### Example 1: Synthesis of the random mutational library

A modified PCR protocol (Svetlov and Cooper, 1998) was used: initial denaturation at 95°C for 3 min, 30 cycles of denaturation at 95°C for 5 s, annealing at 50°C for 5 s, and primer extension at 72°C for 5 s, and final extension at 72°C for 5 min, performed on the Cycler PTC-200 (MJ-Research).

The reaction was carried out with 2.5 U of Taq polymerase (Promega) using standard reaction buffer (10 mM Tris-HCI, pH 8.3, 1.5 mM MgCl₂, 50 mM KCI) supplemented with 0.5 mM MnSO₄. An equimolar mix of dNTPs (Amersham Pharmacia Biotech Inc) was used to provide 500 µM of each nucleotide triphosphate in a 100 µl reaction volume. 10 ng of the p416GPD-Edg5 plasmid were used as template. The following oligonucleotides (30 pmol of each) were used as primers for the PCR amplification: EDG5 fwd CAR (SEQ ID NO. 1: 5'-ATG GGC AGC TTG TAC TCG GAG T-3') and EDG5 rev CAR (SEQ ID NO. 2: 5'-TCA GAA CAC CGT GTT GCC CTC-3'). They correspond exactly to the first 22 and last 21 nucleotides of the receptor's sequence, thus the PCR amplifies exactly the open reading frame.

The PCR product (about 5 µg) was purified by electrophoresis through a 1% agarose-TBE gel followed by elution into 40 µl sterile water (QIAquick Gel Extraction Kit, Qiagen). The DNA final concentration was about 0.1 µg/µl.

### Example 2: In vivo recombination (Gap Repair)

2 µl of the PCR product were cloned into the TA Topo Cloning Vector (Invitrogen) for further qualitative and quantitative analysis of the randomly induced mutations.

The remaining volume of purified PCR product (3-5 µg in 38 µl) was co-transformed with 1 µg of p416GPD-Edg5 (linearized by double digestion with Nhel-Xmal) into about 10⁹ cells of the yeast strain (W303 MATa far1::hisG, sst2::ura3^{FOA}, fus1::HIS3, ΔSte2::Kan^{R}, mfa2-fus1-lacZ::ura3^{FOA}) according to a modified Lithium acetate method (Ito *et al*., 1983).

The transformed yeast cells were plated on 10 plates SC/Glucose -Ura medium to select for the cells with a "repaired plasmid" (about 10⁸ yeast cells per plate).

After 36 hours incubation at 30°C (when very small colonies were visible), the transformation plates were replica-plated onto selective medium: SC/Glucose -Ura-His, pH 6.8, containing 2 mM 3-Aminotriazol (3-AT).

### Example 3: Selection

After 48 hours incubation at 30°C, the colonies still growing were picked and restreaked as patches on a new selective plate (SC/Glucose -Ura -His, pH 6.8, containing 2 mM of 3-AT).

To eliminate false positive mutants (plasmid independent activity), the following steps were performed:

The plasmid from each selected clone was recovered by a Zymolase /SDS treatment protocol adapted from H. Ma & al. (Ma *et al.,* 1987).

After ethanol purification, each plasmid was transformed into *E. coli* DH5α electro-competent cells. Individual bacterial transformants, one for each mutant, were grown in mini culture for plasmid preparation (QIAprep Spin Miniprep Kit, Qiagen).

The purified DNA was then transformed again into the same yeast strain and each mutant assayed.

### Example 4: Solid phase assay

From a 16 hours culture in SC/glucose -Ura medium, about 3x10⁵ cells of each mutant (in triplicates) were spotted onto three different plates:
SC/Glucose -Ura as a control (to make sure that every spot contains roughly the same number of cells);
SC/Glucose -Ura -His, pH 6.8, containing 2 mM 3-AT;
SC/Glucose -Ura, pH 7, containing 100 µg/ml X-Gal (5-Bromo-4-chloro-3-indolyl β-D-galactopyranoside).

The two first plates were analyzed after 48 hours of growth at 30°C; the third one was kept for 2 or 3 additional days at 4°C to develop the blue coloration due to β-Galactosidase activity.

We selected the clones which grew on selective medium (SC/Glucose -Ura -His, pH 6.8, containing 2 mM 3-AT) and gave rise to a blue colored patch on X-Gal medium (SD -Ura, pH 7, containing 100 µg/ml X-Gal). These were candidates for constitutive activity (Figure 4).

### Example 5: Liquid assay in 96 well format

The same 16 hours culture was diluted 200 times in selective medium (SC/Glucose -Ura -His, pH 6.8, containing 2 mM 3-AT) and 90 µl were dispensed into the 8 wells of a microtiter plate column already containing 10 µl of a serial dilution of the ligand Spingosine 1-Phosphate (Matreya) (solubilized and diluted in water from 10⁻³ to 10⁻⁹ M).

After 18 to 24 hours of stimulation/growth in a shaking incubator (700 rpm, 30°C), the β-Galactosidase activity was detected with the substrate Chlorophenolred-β-D-galactopyranoside (CPRG, Boehringer).

### Example 6: Experimental Procedures in HEK 293

The wild type Edg5 receptor and 3 of the 22 CAMs were sub-cloned into the mammalian expression vector pcDNA3.1(+) to be tested in cell culture (Figure 3). A HEK 293 cell line stably transfected with the reporter construct 6SRE-Luciferase was utilized for the assay.
This adherent cell line was grown under normal conditions (37°C, 5% CO₂, humid atmosphere) in DMEM-Glutamax (Gibco BRL) + 1% Penicillin/Streptomycin + 10% Fetal Bovine Serum.

### Example 6.1: Transfection

Day 1- 40.000 cells/well were plated in a white 96-well plate

Day 2- the cells were rinsed with 200µl Opti-MEM (Gibco BRL) and each well received 100µl of a transfection mix containing: 0.5 µg receptor plasmid + 0.25 µg CMV-β Gal (Promega) + 1.2 µl Lipofectamine (Life Technologies) in Opti-MEM.

After 5 hours of incubation with this mix, the wells were emptied and received 180 µl of the normal culture medium (DMEM-Glutamax + 1% Penicillin/Streptomycin) containing only 0.5% of Fetal Bovine Serum.

Day 3- 20 µl of a 10-fold concentrated serial dilution of Sphingosine 1-Phosphate (from 10⁻⁴ to 10⁻¹⁰ M) was added to each well.

Day 4- the wells were emptied, rinsed with 200 µl phosphate buffer (without calcium and magnesium) and received 50 µl of Glo Lysis Buffer (Promega), after 5 minutes at room temperature, they received 50 µl of Steady-Glo Luciferase Reagent, and the measurement was achieved 5 minutes later in a Luminoskan (Labsystem), 15 seconds integration of the signal.

To normalize the results of the assay, β-Galactosidase activity was measured, from the same plate, after 5 minutes incubation with 25 µl of Gal-Screen Reagent (Tropix), 5 seconds integration of the signal. The luciferase numbers were then divided by the β-Galactosidase numbers.

### Example 7: Results

### Example 7.1: TA cloning analysis

The analysis of 38 randomly sequenced clones revealed 28 nucleotide mutations: 5 silent mutations, 1 STOP codon and 22 amino acid substitutions. These results suggest that under the experimental conditions the probability for an amino acid substitution to occur in the 354 residues of the wild-type sequence is 0.61.

### Example 7.2: Analysis of selected mutants

The solid phase assay gives a confirmation of the first selection done after replicaplating the gap repair plates on selection plates. After being grown again on selective plate as patches (for confirmation), the plasmid DNA carrying the active mutant was purified, amplified in *E*. *coli* and re-transformed into the same yeast strain.

Three colonies of each yeast transformation were tested for growth and for β-Galactosidase activity on selective plates. The Figure 4 illustrates the clear response from the different mutants obtained in this assay. Here, mutants 1 (contains two mutations, Ala82Val and Ile197Thr) and 7 (Ala82Val only) look the most active (i.e. fast growth on selective plate and blue coloration on X-Gal plate). Mutants 2 (Thr196lle), 3 (Ser159Pro), 5 (Phe242Leu) and 8 (Ser159Pro and Val215Met) were also selected (at least two of the three clones grew), but appear less active (i.e. the blue coloration is not so obvious). Clone 4 had the same activating Phe242Leu mutation but only one of the three colonies grew. Clone 6 had no activating mutation.

To further characterize the mutants, their activity was tested in a liquid assay. Triplicates of each mutant (re-transformed in yeast) were grown to saturation in a pre-culture. These cell suspensions were diluted 200 times into a medium lacking histidine, permitting the growth of only activated receptors (HIS3 gene under the control of the FUS1 promoter) and distributed in a 96-well microtiterplate together with an increasing concentration of Sphingosine 1-Phosphate. After 24 hours of incubation and shaking at 30°C, the presence of β-Galactosidase activity was measured in a colorimetric assay by adding the substrate CPRG, incubating 2 hours at 30°C and measuring the absorbance at 574nm.

Figure 5 shows that even in the absence of ligand, mutant Ala82Val is hyper-active (which correlates very well with the observations made in the plate assay), while others have a basal activity intermediate between the Ala82Val mutant and the wild type receptor (Ser159Pro and Val238Glu).

Out of three independent screens, 22 mutations have been found to confer constitutive activity to the Edg5 receptor (increased basal activity and the ability to be further stimulated by Sphingosine 1-Phosphate) (Table 1). Interestingly, the mutation Ser159Pro was isolated in each of the three screens, and the mutations Ala82Val and Phe242Leu were isolated in two of the three screens.

### Example 7.3: HEK 293 assay

A Serum Responsive Element (SRE) - Luciferase reporter assay in HEK 293 was chosen to verify in mammalian cells the activity of the CAMs selected with the yeast system. A stable HEK 293 cell line carrying the 6SRE-Luciferase construct was transfected with the wild type Edg5 or the mutants Ala82Val, Ser159Pro and Val238Glu. After 24 hours of stimulation, the measurement of Luciferase reflected the receptor's activity.

This assay (figure 6) shows an increased basal activity (i.e. in absence of agonist) of the three mutants compared to the wild type, although the maximum response of all four receptors (wild type and mutants) was not changed.

### Example 7.4: Systematic screen

To validate a screen, the whole process must be repeated in the same conditions. Indeed, the PCR principle can create an important bias introducing an activating (i.e. the Ala 82 -> Val mutation found 68 times in the third screen) or inactivating mutation in an early stage of the reaction. This mutation is then present in a high percentage of clones and can mask other interesting point mutations. This has to be circumvented. The best and fastest way would be doing at least three low-fidelity PCRs at the time and all the following steps in parallel.

**Table 1:**

| Selected mutants analysis Out of three independent experiments, 22 mutations have been found to confer constitutive activity to the Edg5 receptor: | | | | |
|---|---|---|---|---|
| | First screen (4 active mutants) | Second screen (3 active mutants) | Third screen (118 active mutants) | Location |
| Leu 70 -> Pro | | | 2 | Transmembrane domain 2 |
| Phe 71 -> Leu | | | 7 | '' |
| Ala 82 -> Val | 2 | | 68 | '' |
| Val 87 -> Ala | | | 1 | '' |
| Ser 113 -> Le | | | | Transmembrane domain 3 |
| Leu 139 -> Pro | | | 2 | Intracellular loop 2 |
| Ser 155 -> Pro | | | 1 | Transmembrane domain 4 |
| Ser 159 -> Pro | 1 | 1 | 25 | '' |
| Val 183 -> Ala | | | 2 | Extracellular loop 2 |
| Ala 187 -> Thr | | | 1 | '' |
| Lys 188 -> Arg | | | 1 | '' |
| Thr 196 -> Ile | 1 | | | Transmembrane domain 5 |
| lie 205 -> Phe | | | 3 | '' |
| Leu 229 -> Pro | | | 1 | Transmembrane domain 6 |
| Leu 232 -> Arg | | | 3 | '' |
| Thr 234 -> Ala | | | 4 | '' |
| Val 235 -> lie | | | 4 | '' |
| Thr 236 -> lie | | | 1 | '' |
| Val 238 -> Glu | | | 2 | '' |
| Val 238 -> Ala | | | 3 | '' |
| Phe 242 -> Leu | | 2 | 1 | '' |
| Phe 250 -> Tyr | | | 2 | '' |

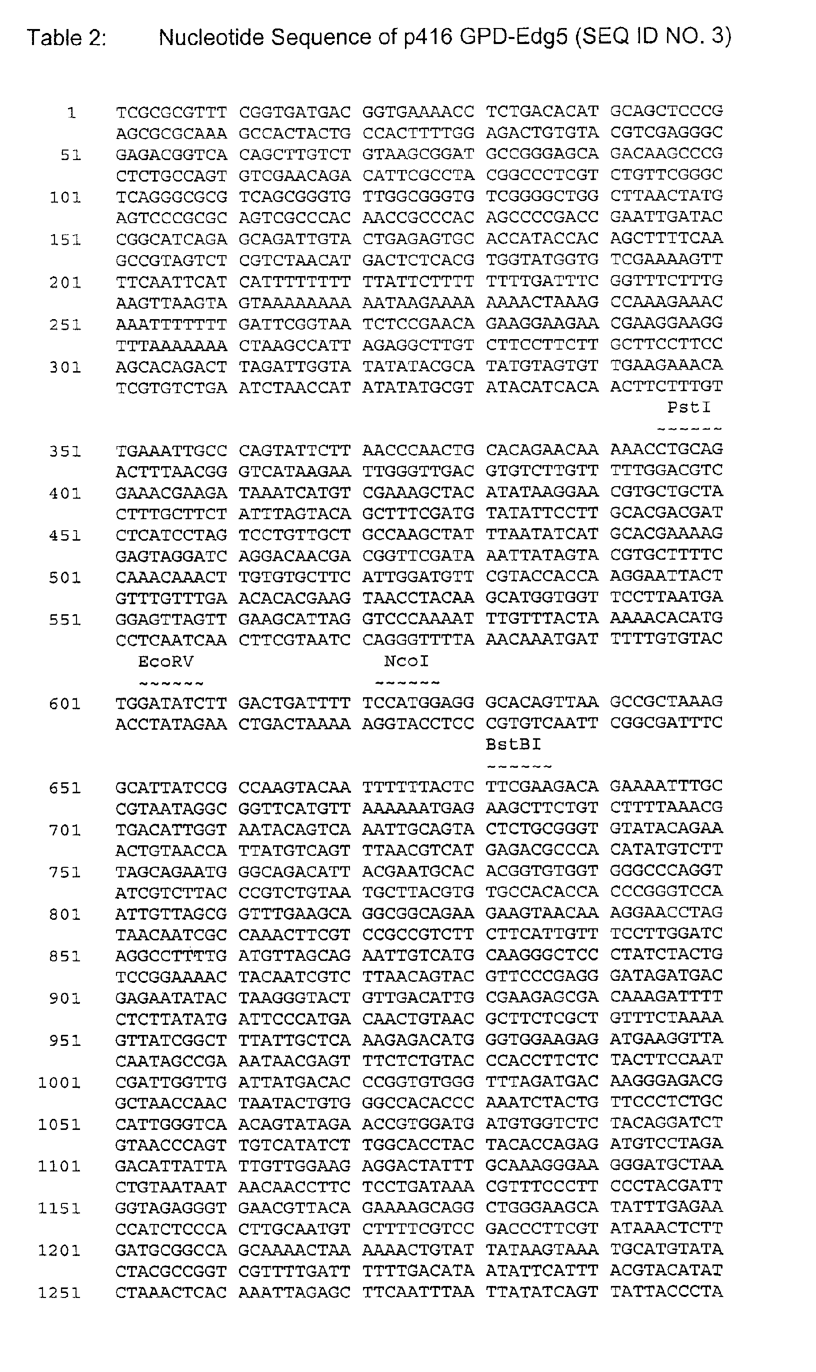

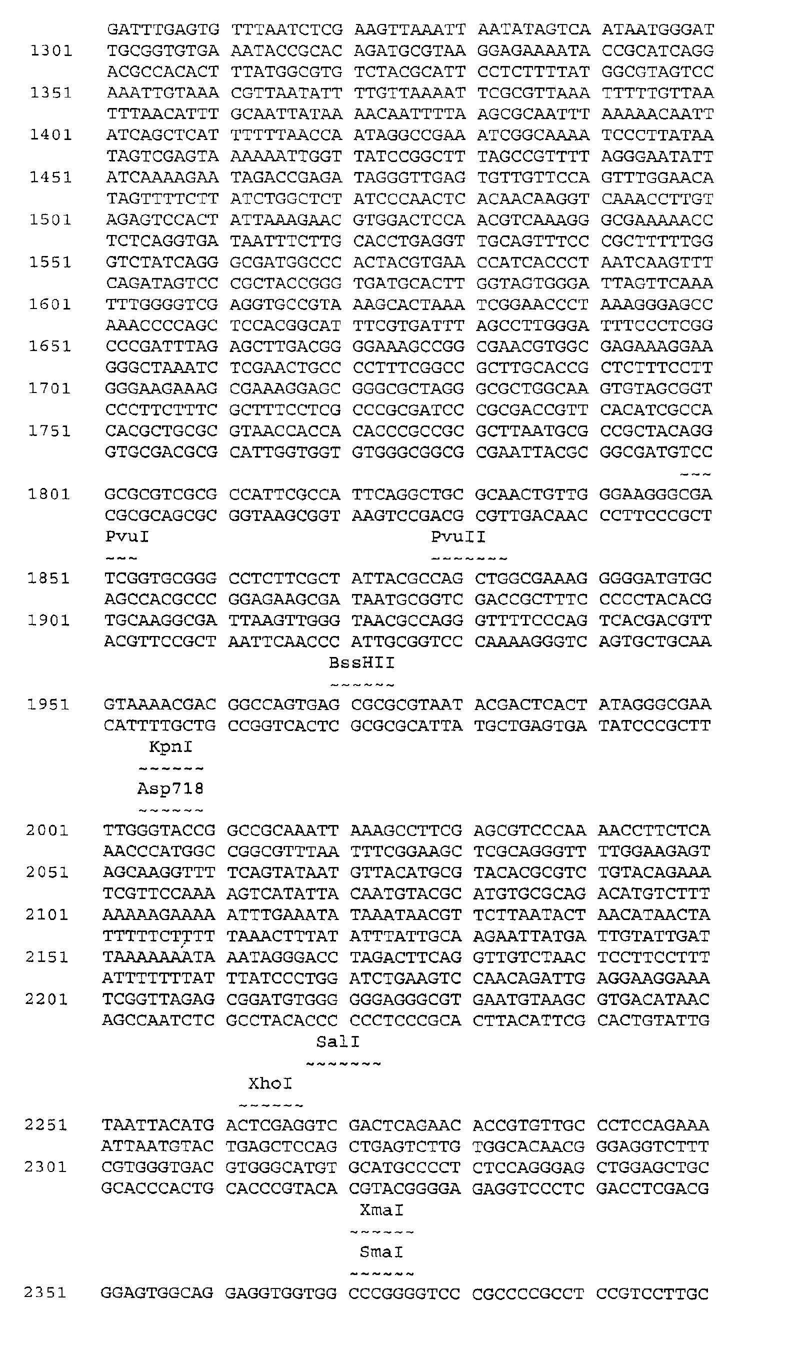

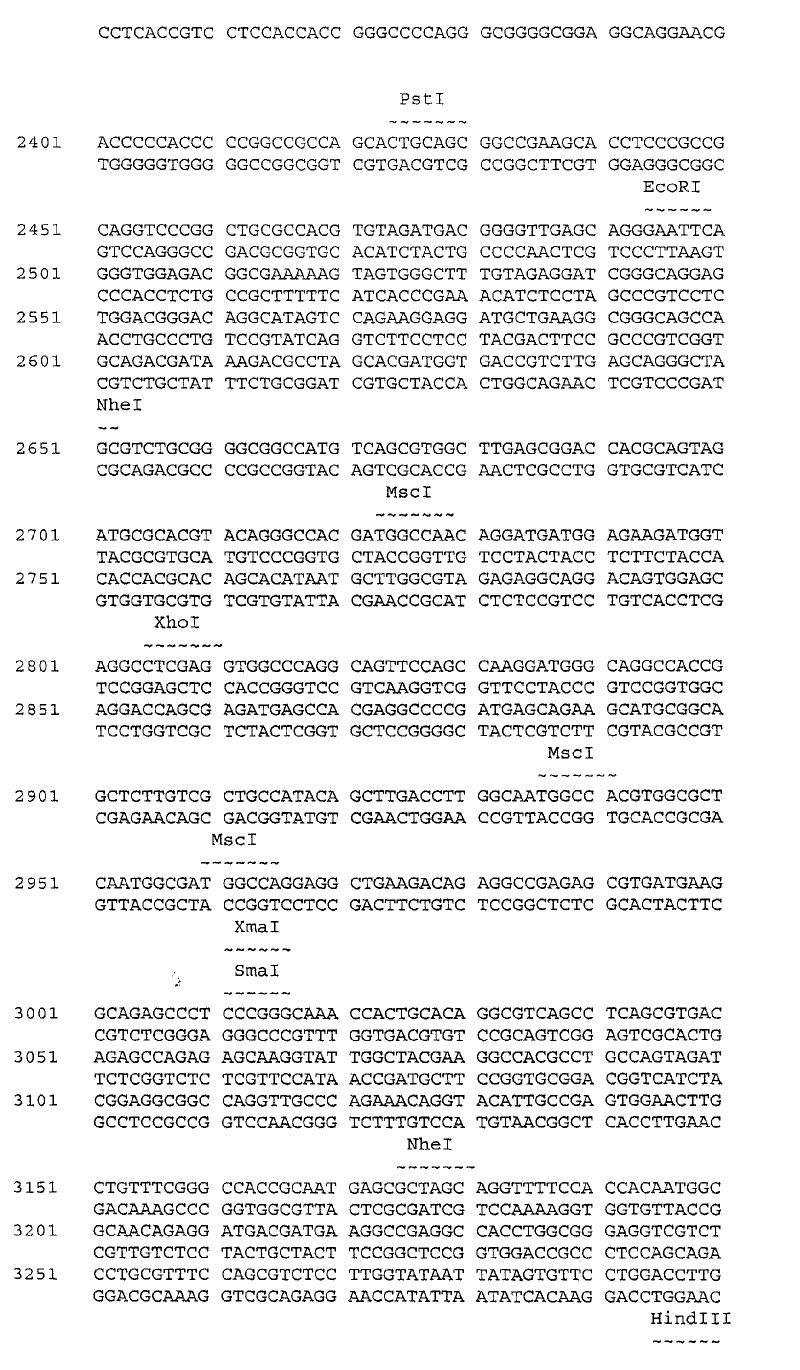

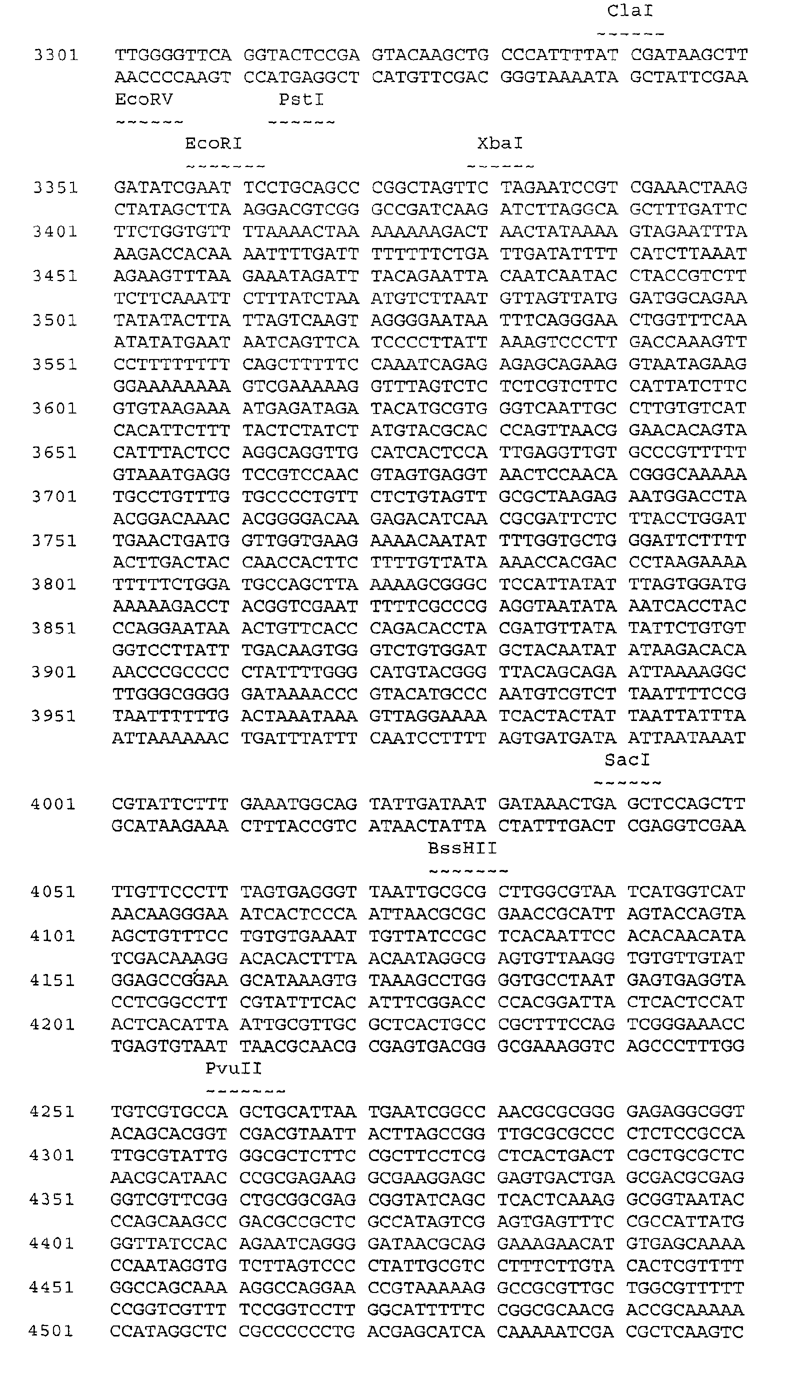

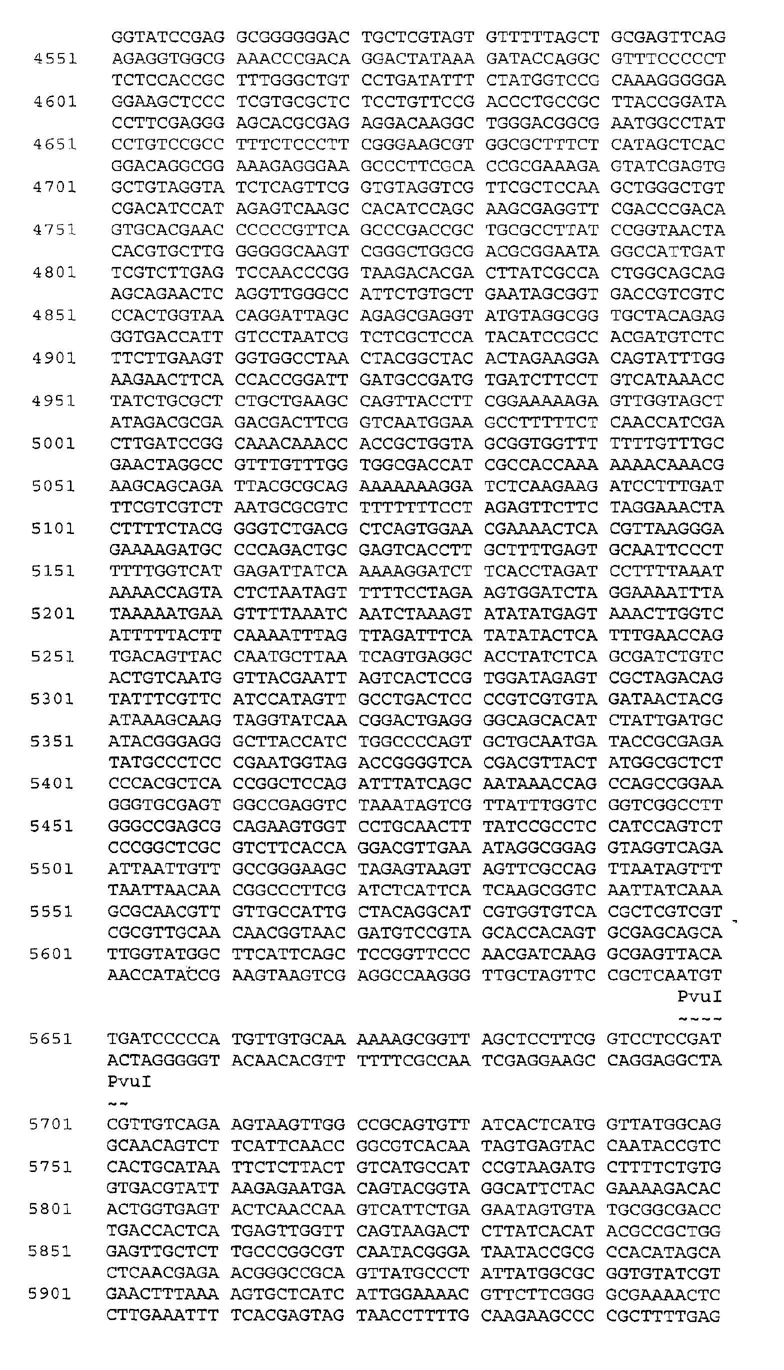

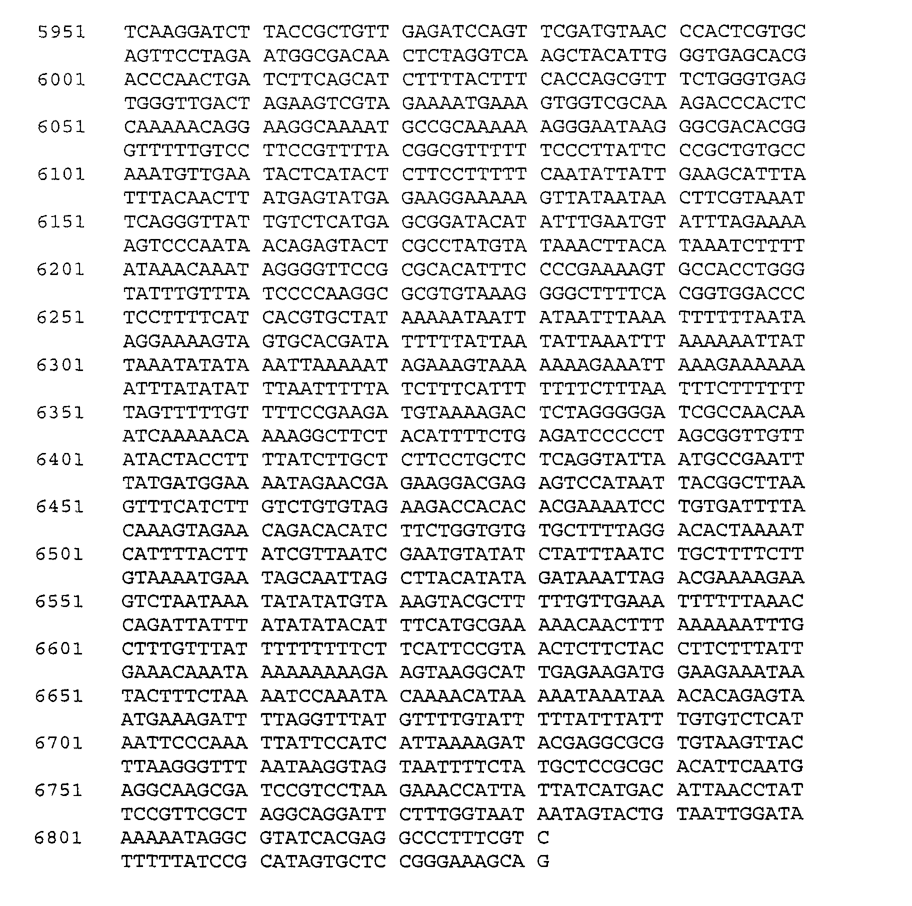

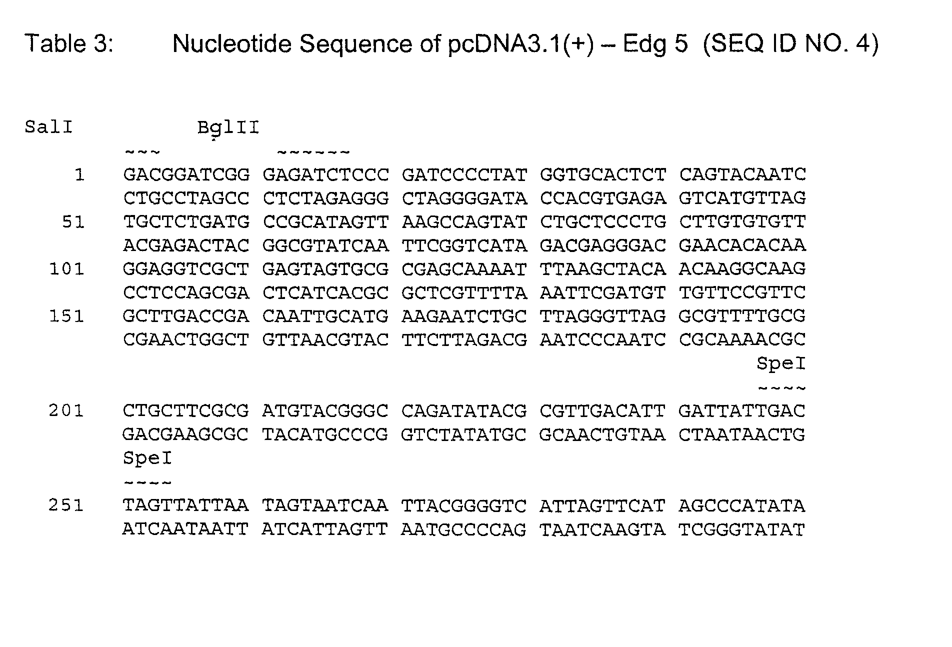

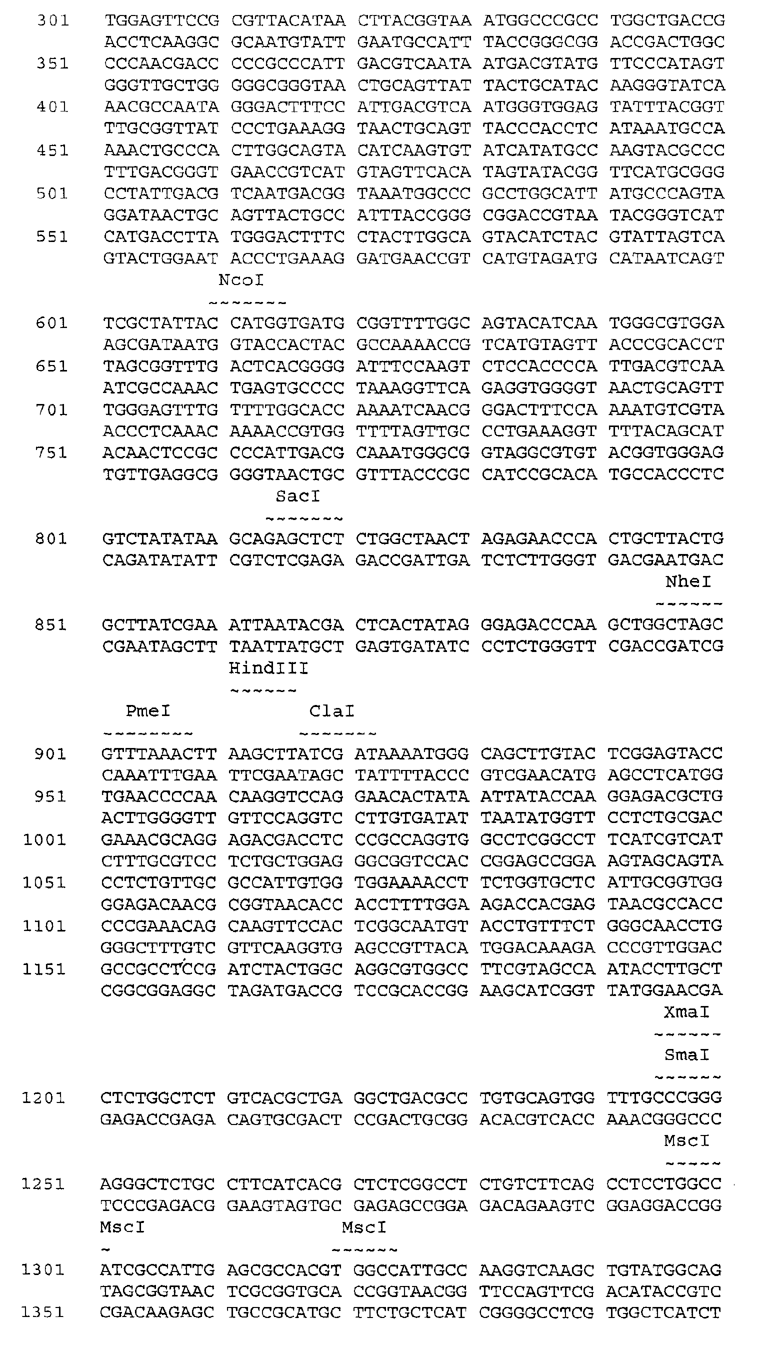

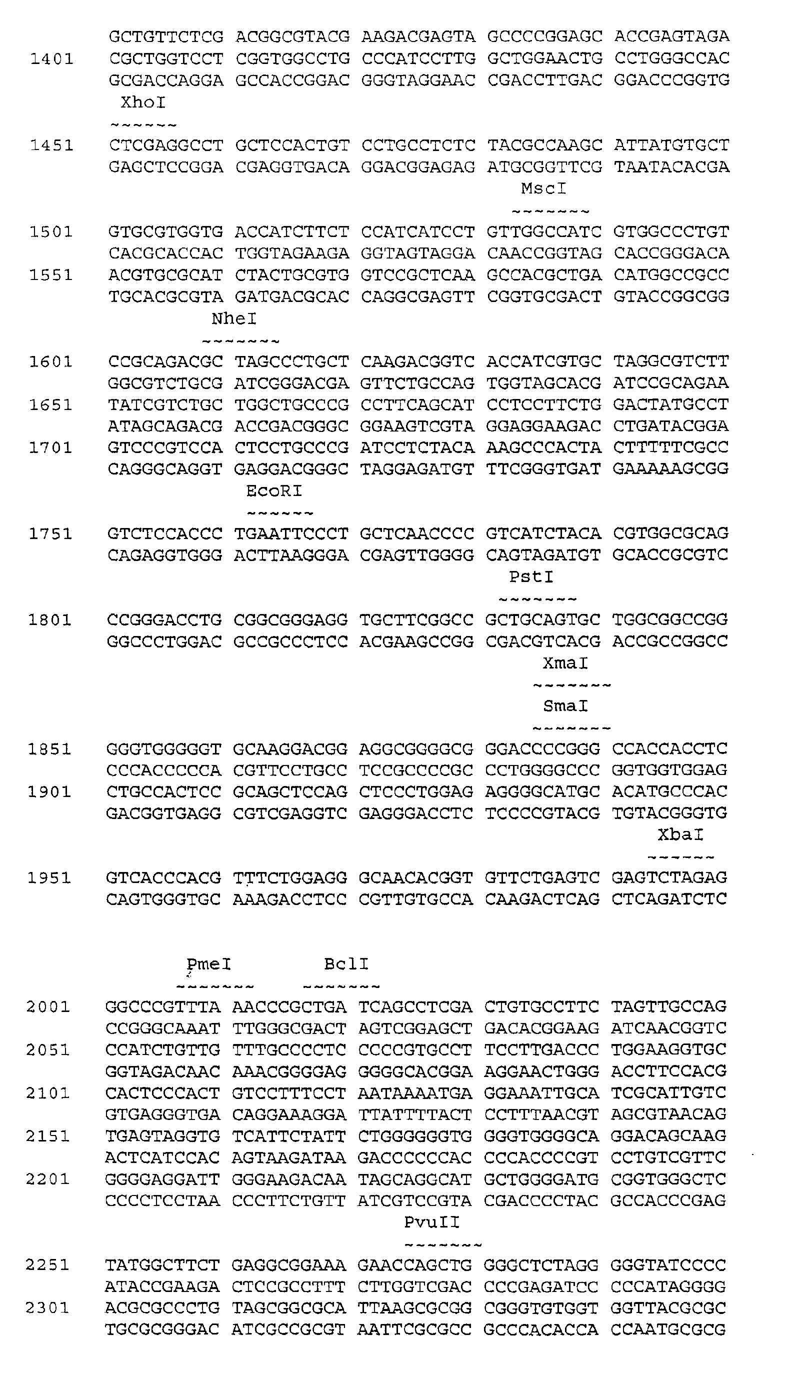

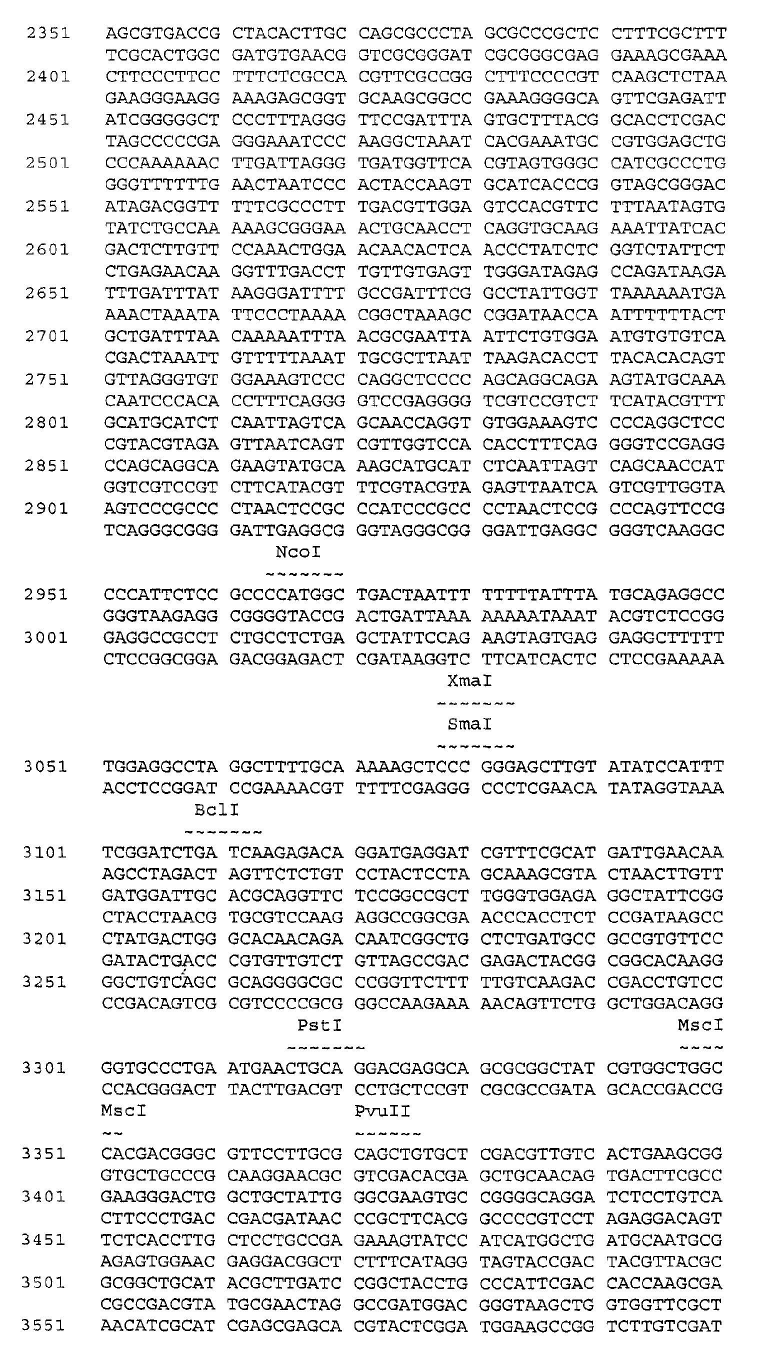

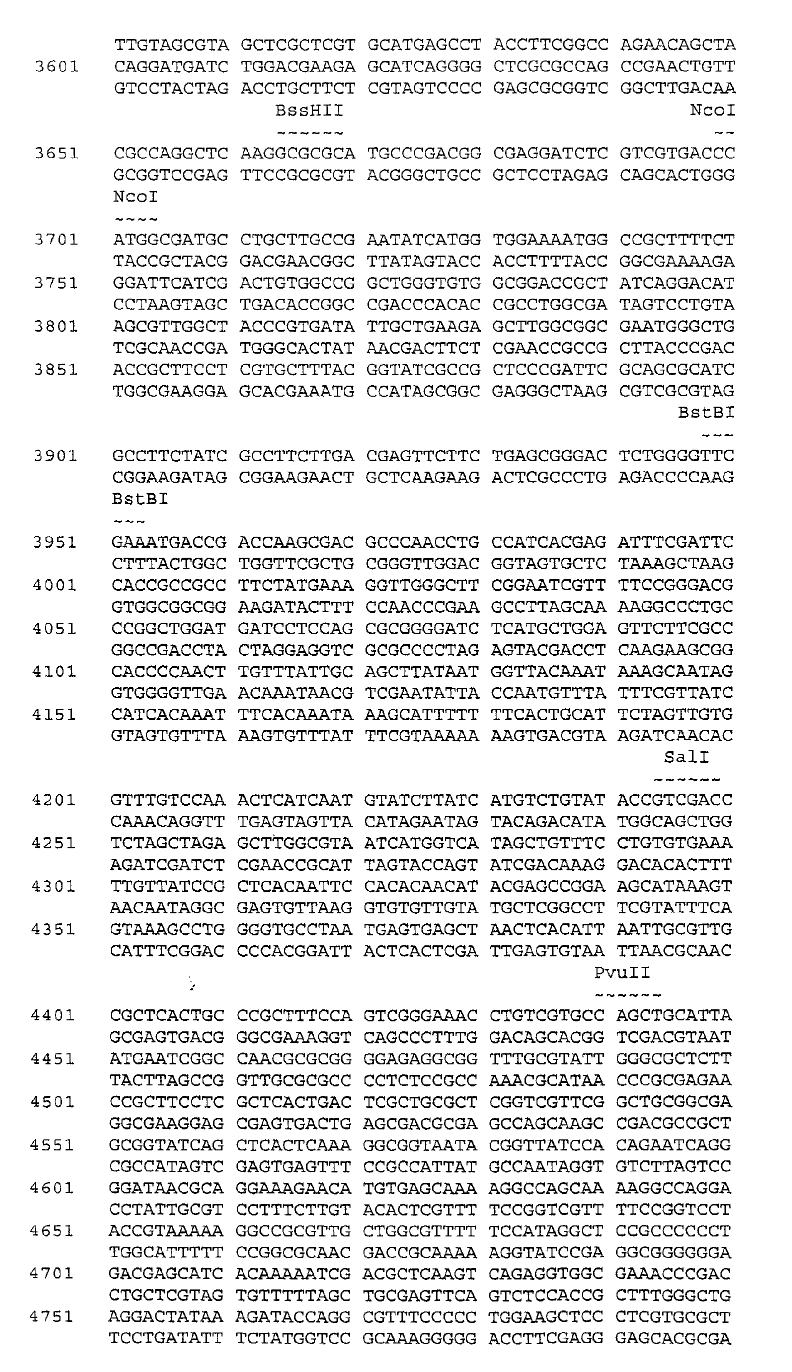

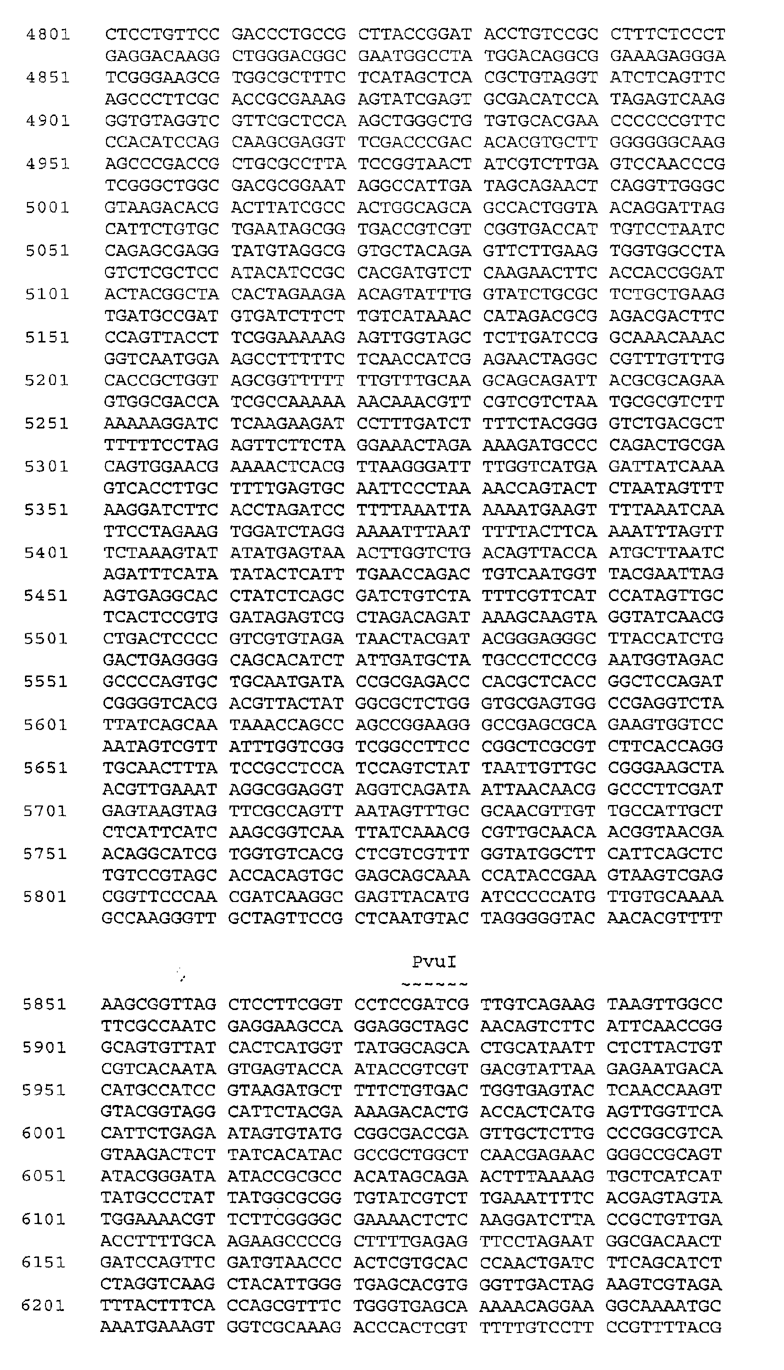

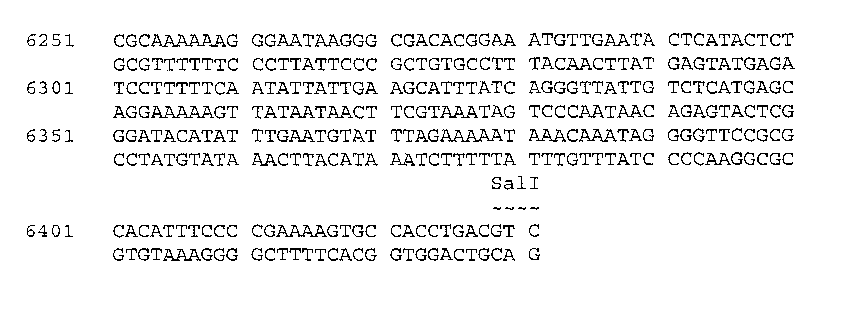

### Reference List

Alewijnse AE, Timmerman H, Jacobs E H, Smit M J, Roovers E, Cotecchia S and Leurs R (2000) The Effect of Mutations in the DRY Motif on the Constitutive Activity and Structural Instability of the Histamine H₂ Receptor. *Mol Pharmacol* **57**: pp 890-898.

An S, Zheng Y and Bleu T (2000) Sphingosine 1-Phosphate-Induced Cell Proliferation, Survival, and Related Signaling Events Mediated by G Protein-Coupled Receptors Edg3 and Edg5. *J Biol Chem* **275**: pp 288-296.

Botstein D, Chervitz S A and Cherry J M (1997) Yeast As a Model Organism. *Science* **277**: pp 1259-1260.

Brown AJ, Dyos S L, Whiteway M S, White J H, Watson M A, Marzioch M, Clare J J, Cousens D J, Paddon C, Plumpton C, Romanos M A and Dowell S J (2000) Functional Coupling of Mammalian Receptors to the Yeast Mating Pathway Using Novel Yeast/Mammalian G Protein α-Subunit Chimeras. *Yeast* **16**: pp 11-22.

Chambers JK, Macdonald L E, Sarau H M, Ames R S, Freeman K, Foley J J, Zhu Y, McLaughlin M M, Murdock P, McMillan L, Trill J, Swift A, Aiyar N, Taylor P, Vawter L, Naheed S, Szekeres P, Hervieu G, Scott C, Watson J M, Murphy A J, Duzic E, Klein C, Bergsma D J, Wilson S and Livi G P (2000) A G Protein-Coupled Receptor for UDP-Glucose. *J Biol Chem* **275**: pp 10767-10771.

Chen G, Way J, Armour S, Watson C, Queen K, Jayawickreme C K, Chen W J and Kenakin T (2000) Use of Constitutive G Protein-Coupled Receptor Activity for Drug Discovery. *Mol Pharmacol* **57**: pp 125-134.

Daeffler L and Landry Y (2000) Inverse Agonism at Heptahelical Receptors: Concept, Experimental Approach and Therapeutic Potential. *Fundam Clin Pharmacol* **14**: pp 73-87.

Dhanasekaran N, Heasley L E and Johnson G L (1995) G Protein-Coupled Receptor Systems Involved in Cell Growth and Oncogenesis. *Endocr Rev* **16**: pp 259-270.

Duprez L, Parma J, Costagliola S, Hermans J, Van Sande J, Dumont J E and Vassart G (1997) Constitutive Activation of the TSH Receptor by Spontaneous Mutations Affecting the N-Terminal Extracellular Domain. *FEES Lett* **409**: pp 469-474.

Egan CT, Herrick-Davis K and Teitler M (1998) Creation of a Constitutively Activated State of the 5-Hydroxytryptamine2A Receptor by Site-Directed Mutagenesis: Inverse Agonist Activity of Antipsychotic Drugs. *J Pharmacol Exp Ther* **286**: pp 85-90.

Erlenbach I, Kostenis E, Schmidt C, Serradeil-Le Gal C, Raufaste D, Dumont M E, Pausch M H and Wess J (2001) Single Amino Acid Substitutions and Deletions That Alter the G Protein Coupling Properties of the V2 Vasopressin Receptor Identified In Yeast by Receptor Random Mutagenesis. *J Biol Chem* pp M103203200.

Hadcock JR and Pausch M (1999) Ligand screening of G protein-coupled receptors in yeast, in G *Protein-Coupled Receptors* (Haga T and Berstein G eds) pp 49-69, CRC Press LLC, Boca Raton, Florida.

Hla T (2001) Sphingosine 1-Phosphate Receptors. *Prostaglandins & Other Lipid Mediators* **64**: pp 135-142.

Ito H, Fukuda Y, Murata K and Kimura A (1983) Transformation of Intact Yeast Cells Treated With Alkali Cations. *J Bacteriol* **153**: pp 163-168.

Jensen AA, Spalding T A, Burstein E S, Sheppard P O, O'Hara P J, Brann M R, Krogsgaard-Larsen P and Bräuner-Osborne H (2000) Functional Importance of the Ala¹¹⁶-Pro¹³⁶ Region in the Calcium-Sensing Receptor. *J Biol Chem* **275**: pp 29547-29555.

Konopka JB, Margarit S M and Dube P (1996) Mutation of Pro-258 in Transmembrane Domain 6 Constitutively Activates the G Protein-Coupled α-Factor Receptor. *Proc Natl Acad Sci U S A* **93**: pp 6764-6769.

Lefkowitz RJ, Cotecchia S, Samama P and Costa T (1993) Constitutive Activity of Receptors Coupled to Guanine Nucleotide Regulatory Proteins. *Trends Pharmacol Sci* **14**: pp 303-307.

Ma H, Kunes S, Schatz P J and Botstein D (1987) Plasmid Construction by Homologous Recombination in Yeast. *Gene* **58**: pp 201-216.

MacEwan DJ and Milligan G (1996) Inverse Agonist-Induced Up-Regulation of the Human B₂-Adrenoceptor in Transfected Neuroblastoma X Glioma Hybrid Cells. *Mol Pharmacol* **50**: pp 1479-1486.

Oldenburg KR, Vo K T, Michaelis S and Paddon C (1997) Recombination-Mediated PCR-Directed Plasmid Construction *in Vivo* in Yeast. *Nucleic Acids Res* **25**: pp 451-452.

Parnot C, Bardin S, Miserey-Lenkei S, Guedin D, Corvol P and Clauser E (2000) Systematic Identification of Mutations That Constitutively Activate the Angiotensin II Type 1A Receptor by Screening a Randomly Mutated CDNA Library With an Original Pharmacological Bioassay. *Proc Natl Acad Sci U S A* **97**: pp 7615-7620.

Price LA, Kajkowski E M, Hadcock J R, Ozenberger B A and Pausch M H (1995) Functional Coupling of a Mammalian Somatostatin Receptor to the Yeast Pheromone Response Pathway. *Mol Cell Biol* **15**: pp 6188-6195.

Rao VR and Oprian D D (1996) Activating Mutations of Rhodopsin and Other G Protein-Coupled Receptors. *Annu Rev Biophys Biomol Struct* **25**: pp 287-314.

Robinson PR, Cohen G B, Zhukovsky E A and Oprian D D (1992) Constitutively Active Mutants of Rhodopsin. *Neuron* **9**: pp 719-725.

Samama P, Bond R A, Rockman H A, Milano C A and Lefkowitz R J (1997) Ligand-Induced Overexpression of a Constitutively Active B₂-Adrenergic Receptor:

Pharmacological Creation of a Phenotype in Transgenic Mice. *Proc Natl Acad Sci U S A* **94**: pp 137-141.

Sommers CM and Dumont M E (1997) Genetic Interactions Among the Transmembrane Segments of the G Protein Coupled Receptor Encoded by the Yeast STE2 Gene. *J Mol Biol* **266**: pp 559-575.

Sommers CM, Martin N P, Akal-Strader A, Becker J M, Naider F and Dumont M E (2000) A Limited Spectrum of Mutations Causes Constitutive Activation of the Yeast Alpha-Factor Receptor [In Process Citation]. *Biochemistry* **39**: pp 6898-6909.

Spiegel AM (1996) Mutations in G Proteins and G Protein-Coupled Receptors in Endocrine Disease. J *Clin Endocrinol Metab* **81**: pp 2434-2442.

Svetlov V and Cooper T G (1998) Efficient PCR-Based Random Mutagenesis of Sub-Genic (100 Bp) DNA Fragments. *Yeast* **14**: pp 89-91.

Tate CG and Grisshammer R (1996) Heterologous Expression of G-Protein-Coupled Receptors. *Trends Biotechnol* **14**: pp 426-430.

Tsao P, Cao T and von Zastrow M (2001) Role of Endocytosis in Mediating Downregulation of G-Protein-Coupled Receptors. *Trends Pharmacol Sci* **22**: pp 91-96.

Varma DR, Shen H, Deng X F, Peri K G, Chemtob S and Mulay S (1999) Inverse Agonist Activities of B-Adrenoceptor Antagonists in Rat Myocardium. *Br J Pharmacol* **127**: pp 895-902.

Zwick E, Hackel P O, Prenzel N and Ullrich A (1999) The EGF Receptor As Central Transducer of Heterologous Signalling Systems. *Trends Pharmacol Sci* **20**: pp 408-412.

## Claims

1. Method of identifying protein CAMs (constitutively active mutants) wherein
a) a library of mutated sequences of a protein is generated,
b) yeast cells are transformed with such library, and
c) the respective protein CAM is identified.

2. Method of identifying protein CAMs (constitutively active mutants) wherein
d) a library of mutated sequences of a protein is generated,
e) yeast cells are co-transformed with the library and a linearized expression vector,
f) the transformed yeast cells are selected for the repair of the plasmid, and
g) protein CAMs are identified by determining the activity of the respective protein mutant.

3. Method as claimed in claim 1 or 2, wherein the protein is a GPCR (G-Protein coupled receptor), an ion-channel or an enzyme.

4. Method as claimed in claim 3, wherein the enzyme is a kinase.

5. Method as claimed in one of the foregoing claims, wherein the protein is a mammalian protein.

6. Use of the method as claimed in claims 1 to 5, for identifying agonists or inverse agonists.


SEQUENCE LISTING

<110> Aventis Pharma Deutschland GmbH

<120> Method of identifying protein CAMs (Constitutively
      active mutants)

<130> DEAV2002/0003

<140> 02000733.2
<141> 2002-01-12

<160> 4

<170> PatentIn Ver. 2.1

<210> 1
<211> 22
<212> DNA
<213> Artificial Sequence

<220>
<223> Description of Artificial Sequence:Oligonucleotid

<220>
<221> misc_structure
<222> (1)..(22)

<400> 1
atgggcagct tgtactcgga gt                                          22

<210> 2
<211> 21
<212> DNA
<213> Artificial Sequence

<220>
<223> Description of Artificial Sequence:Oligonucleotid

<220>
<221> misc_feature
<222> (1)..(21)

<400> 2
tcagaacacc gtgttgccct c                                           21

<210> 3
<211> 13738
<212> DNA
<213> Artificial Sequence

<220>
<223> Description of Artificial Sequence:Recombinantes
      Plasmid

<220>
<221> misc_feature
<222> (1)..(13738)

<400> 3
tcgcgcgttt cggtgatgac ggtgaaaacc tctgacacat gcagctcccg agcgcgcaaa 60
gccactactg ccacttttgg agactgtgta cgtcgagggc gagacggtca cagcttgtct 120
gtaagcggat gccgggagca gacaagcccg ctctgccagt gtcgaacaga cattcgccta 180
cggccctcgt ctgttcgggc tcagggcgcg tcagcgggtg ttggcgggtg tcggggctgg 240
cttaactatg agtcccgcgc agtcgcccac aaccgcccac agccccgacc gaattgatac 300
cggcatcaga gcagattgta ctgagagtgc accataccac agcttttcaa gccgtagtct 360
cgtctaacat gactctcacg tggtatggtg tcgaaaagtt ttcaattcat catttttttt 420
ttattctttt ttttgatttc ggtttctttg aagttaagta gtaaaaaaaa aataagaaaa 480
aaaactaaag ccaaagaaac aaattttttt gattcggtaa tctccgaaca gaaggaagaa 540
cgaaggaagg tttaaaaaaa ctaagccatt agaggcttgt cttccttctt gcttccttcc 600
agcacagact tagattggta tatatacgca tatgtagtgt tgaagaaaca tcgtgtctga 660
atctaaccat atatatgcgt atacatcaca acttctttgt sttgaaattg cccagtattc 720
ttaacccaac tgcacagaac aaaaacctgc agactttaac gggtcataag aattgggttg 780
acgtgtcttg tttttggacg tcgaaacgaa gataaatcat gtcgaaagct acatataagg 840
aacgtgctgc tactttgctt ctatttagta cagctttcga tgtatattcc ttgcacgacg 900
atctcatcct agtcctgttg ctgccaagct atttaatatc atgcacgaaa aggagtagga 960
tcaggacaac gacggttcga taaattatag tacgtgcttt tccaaacaaa cttgtgtgct 1020
tcattggatg ttcgtaccac caaggaatta ctgtttgttt gaacacacga agtaacctac 1080
aagcatggtg gttccttaat gaggagttag ttgaagcatt aggtcccaaa atttgtttac 1140
taaaaacaca tgcctcaatc aacttcgtaa tccagggttt taaacaaatg atttttgtgt 1200
accrvnctgg atatcttgac tgatttttcc atggagggca cagttaagcc gctaaagacc 1260
tatagaactg actaaaaagg tacctcccgt gtcaattcgg cgatttcbst bgcattatcc 1320
gccaagtaca attttttact cttcgaagac agaaaatttg ccgtaatagg cggttcatgt 1380
taaaaaatga gaagcttctg tcttttaaac gtgacattgg taatacagtc aaattgcagt 1440
actctgcggg tgtatacaga aactgtaacc attatgtcag tttaacgtca tgagacgccc 1500
acatatgtct ttagcagaat gggcagacat tacgaatgca cacggtgtgg tgggcccagg 1560
tatcgtctta cccgtctgta atgcttacgt gtgccacacc acccgggtcc aattgttagc 1620
ggtttgaagc aggcggcaga agaagtaaca aaggaaccta gtaacaatcg ccaaacttcg 1680
tccgccgtct tcttcattgt ttccttggat caggcctttt gatgttagca gaattgtcat 1740
gcaagggctc cctatctact gtccggaaaa ctacaatcgt cttaacagta cgttcccgag 1800
ggatagatga cgagaatata ctaagggtac tgttgacatt gcgaagagcg acaaagattt 1860
tctcttatat gattcccatg acaactgtaa cgcttctcgc tgtttctaaa agttatcggc 1920
tttattgctc aaagagacat gggtggaaga gatgaaggtt acaatagccg aaataacgag 1980
tttctctgta cccaccttct ctacttccaa tcgattggtt gattatgaca cccggtgtgg 2040

gtttagatga caagggagac ggctaaccaa ctaatactgt gggccacacc caaatctact 2100
gttccctctg ccattgggtc aacagtatag aaccgtggat gatgtggtct ctacaggatc 2160
tgtaacccag ttgtcatatc ttggcaccta ctacaccaga gatgtcctag agacattatt 2220
attgttggaa gaggactatt tgcaaaggga agggatgcta actgtaataa taacaacctt 2280
ctcctgataa acgtttccct tccctacgat tggtagaggg tgaacgttac agaaaagcag 2340
gctgggaagc atatttgaga accatctccc acttgcaatg tcttttcgtc cgacccttcg 2400
tataaactct tgatgcggcc agcaaaacta aaaaactgta ttataagtaa atgcatgtat 2460
actacgccgg tcgttttgat tttttgacat aatattcatt tacgtacata tctaaactca 2520
caaattagag cttcaattta attatatcag ttattaccct agatttgagt gtttaatctc 2580
gaagttaaat taatatagtc aataatggga ttgcggtgtg aaataccgca cagatgcgta 2640
aggagaaaat accgcatcag gacgccacac tttatggcgt gtctacgcat tcctctttta 2700
tggcgtagtc caaattgtaa acgttaatat tttgttaaaa ttcgcgttaa atttttgtta 2760
atttaacatt tgcaattata aaacaatttt aagcgcaatt taaaaacaat tatcagctca 2820
ttttttaacc aataggccga aatcggcaaa atcccttata atagtcgagt aaaaaattgg 2880
ttatccggct ttagccgttt tagggaatat tatcaaaaga atagaccgag atagggttga 2940
gtgttgttcc agtttggaac atagttttct tatctggctc tatcccaact cacaacaagg 3000
tcaaaccttg tagagtccac tattaaagaa cgtggactcc aacgtcaaag ggcgaaaaac 3060
ctctcaggtg ataatttctt gcacctgagg ttgcagtttc ccgctttttg ggtctatcag 3120
ggcgatggcc cactacgtga accatcaccc taatcaagtt tcagatagtc ccgctaccgg 3180
gtgatgcact tggtagtggg attagttcaa atttggggtc gaggtgccgt aaagcactaa 3240
atcggaaccc taaagggagc caaaccccag ctccacggca tttcgtgatt tagccttggg 3300
atttccctcg gcccgattta gagcttgacg gggaaagccg gcgaacgtgg cgagaaagga 3360
agggctaaat ctcgaactgc ccctttcggc cgcttgcacc gctctttcct tgggaagaaa 3420
gcgaaaggag cgggcgctag ggcgctggca agtgtagcgg tcccttcttt cgctttcctc 3480
gcccgcgatc ccgcgaccgt tcacatcgcc acacgctgcg cgtaaccacc acacccgccg 3540
cgcttaatgc gccgctacag ggtgcgacgc gcattggtgg tgtgggcggc gcgaattacg 3600
cggcgatgtc cgcgcgtcgc gccattcgcc attcaggctg cgcaactgtt gggaagggcg 3660
acgcgcagcg cggtaagcgg taagtccgac gcgttgacaa cccttcccgc tvvtcggtgc 3720
gggcctcttc gctattacgc cagctggcga aagggggatg tgcagccacg cccggagaag 3780
cgataatgcg gtcgaccgct ttccccctac acgtgcaagg cgattaagtt gggtaacgcc 3840
agggttttcc cagtcacgac gttacgttcc gctaattcaa cccattgcgg tcccaaaagg 3900
gtcagtgctg caabsshgta aaacgacggc cagtgagcgc gcgtaatacg actcactata 3960
gggcgaacat tttgctgccg gtcactcgcg cgcattatgc tgagtgatat cccgcttkna 4020
sttgggtacc ggccgcaaat taaagccttc gagcgtccca aaaccttctc aaacccatgg 4080
ccggcgttta atttcggaag ctcgcagggt tttggaagag tagcaaggtt ttcagtataa 4140
tgttacatgc gtacacgcgt ctgtacagaa atcgttccaa aagtcatatt acaatgtacg 4200
catgtgcgca gacatgtctt taaaaagaaa aatttgaaat ataaataacg ttcttaatac 4260
taacataact atttttcttt ttaaacttta tatttattgc aagaattatg attgtattga 4320
ttaaaaaaat aaatagggac ctagacttca ggttgtctaa ctccttcctt tattttttta 4380
tttatccctg gatctgaagt ccaacagatt gaggaaggaa atcggttaga gcggatgtgg 4440
ggggagggcg tgaatgtaag cgtgacataa cagccaatct cgcctacacc cccctcccgc 4500
acttacattc gcactgtatt gsahtaatta catgactcga ggtcgactca gaacaccgtg 4560
ttgccctcca gaaaattaat gtactgagct ccagctgagt cttgtggcac aacgggaggt 4620
ctttcgtggg tgacgtgggc atgtgcatgc ccctctccag ggagctggag ctgcgcaccc 4680
actgcacccg tacacgtacg gggagaggtc cctcgacctc gacgmasmag gagtggcagg 4740
aggtggtggc ccggggtccc gccccgcctc cgtccttgcc ctcaccgtcc tccaccaccg 4800
ggccccaggg cggggcggag gcaggaacgs tacccccacc cccggccgcc agcactgcag 4860
cggccgaagc acctcccgcc gtgggggtgg gggccggcgg tcgtgacgtc gccggcttcg 4920

tggagggcgg ccrcaggtcc cggctgcgcc acgtgtagat gacggggttg agcagggaat 4980
tcagtccagg gccgacgcgg tgcacatcta ctgccccaac tcgtccctta agtgggtgga 5040
gacggcgaaa aagtagtggg ctttgtagag gatcgggcag gagcccacct ctgccgcttt 5100
ttcatcaccc gaaacatctc ctagcccgtc ctctggacgg gacaggcata gtccagaagg 5160
aggatgctga aggcgggcag ccaacctgcc ctgtccgtat caggtcttcc tcctacgact 5220
tccgcccgtc ggtgcagacg ataaagacgc ctagcacgat ggtgaccgtc ttgagcaggg 5280
ctacgtctgc tatttctgcg gatcgtgcta ccactggcag aactcgtccc gatnhgcgtc 5340
tgcggggcgg ccatgtcagc gtggcttgag cggaccacgc agtagcgcag acgccccgcc 5400
ggtacagtcg caccgaactc gcctggtgcg tcatcmscat gcgcacgtac agggccacga 5460
tggccaacag gatgatggag aagatggtta cgcgtgcatg tcccggtgct accggttgtc 5520
ctactacctc ttctaccaca ccacgcacag cacataatgc ttggcgtaga gaggcaggac 5580
agtggagcgt ggtgcgtgtc gtgtattacg aaccgcatct ctccgtcctg tcacctcgha 5640
ggcctcgagg tggcccaggc agttccagcc aaggatgggc aggccaccgt ccggagctcc 5700
accgggtccg tcaaggtcgg ttcctacccg tccggtggca ggaccagcga gatgagccac 5760
gaggccccga tgagcagaag catgcggcat cctggtcgct ctactcggtg ctccggggct 5820
actcgtcttc gtacgccgtm scgctcttgt cgctgccata cagcttgacc ttggcaatgg 5880
ccacgtggcg ctcgagaaca gcgacggtat gtcgaactgg aaccgttacc ggtgcaccgc 5940
gamsccaatg gcgatggcca ggaggctgaa gacagaggcc gagagcgtga tgaaggttac 6000
cgctaccggt cctccgactt ctgtctccgg ctctcgcact acttcmasma gcagagccct 6060
cccgggcaaa ccactgcaca ggcgtcagcc tcagcgtgac cgtctcggga gggcccgttt 6120
ggtgacgtgt ccgcagtcgg agtcgcactg agagccagag agcaaggtat tggctacgaa 6180
ggccacgcct gccagtagat tctcggtctc tcgttccata accgatgctt ccggtgcgga 6240
cggtcatcta cggaggcggc caggttgccc agaaacaggt acattgccga gtggaacttg 6300
gcctccgccg gtccaacggg tctttgtcca tgtaacggct caccttgaac nhctgtttcg 6360
ggccaccgca atgagcgcta gcaggttttc caccacaatg gcgacaaagc ccggtggcgt 6420
tactcgcgat cgtccaaaag gtggtgttac cggcaacaga ggatgacgat gaaggccgag 6480
gccacctggc gggaggtcgt ctcgttgtct cctactgcta cttccggctc cggtggaccg 6540
ccctccagca gacctgcgtt tccagcgtct ccttggtata attatagtgt tcctggacct 6600
tgggacgcaa aggtcgcaga ggaaccatat taatatcaca aggacctgga achndcattg 6660
gggttcaggt actccgagta caagctgccc attttatcga taagcttaac cccaagtcca 6720
tgaggctcat gttcgacggg taaaatagct attcgaacrv stcrbagata tcgaattcct 6780
gcagcccggc tagttctaga atccgtcgaa actaagctat agcttaagga cgtcgggccg 6840
atcaagatct taggcagctt tgattcttct ggtgttttaa aactaaaaaa aagactaact 6900
ataaaagtag aatttaaaga ccacaaaatt ttgatttttt ttctgattga tattttcatc 6960
ttaaatagaa gtttaagaaa tagatttaca gaattacaat caatacctac cgtctttctt 7020
caaattcttt atctaaatgt cttaatgtta gttatggatg gcagaatata tacttattag 7080
tcaagtaggg gaataatttc agggaactgg tttcaaatat atgaataatc agttcatccc 7140
cttattaaag tcccttgacc aaagttcctt ttttttcagc tttttccaaa tcagagagag 7200
cagaaggtaa tagaagggaa aaaaaagtcg aaaaaggttt agtctctctc gtcttccatt 7260
atcttcgtgt aagaaaatga gatagataca tgcgtgggtc aattgccttg tgtcatcaca 7320
ttcttttact ctatctatgt acgcacccag ttaacggaac acagtacatt tactccaggc 7380
aggttgcatc actccattga ggttgtgccc gtttttgtaa atgaggtccg tccaacgtag 7440
tgaggtaact ccaacacggg caaaaatgcc tgtttgtgcc cctgttctct gtagttgcgc 7500
taagagaatg gacctaacgg acaaacacgg ggacaagaga catcaacgcg attctcttac 7560
ctggattgaa ctgatggttg gtgaagaaaa caatattttg gtgctgggat tcttttactt 7620
gactaccaac cacttctttt gttataaaac cacgacccta agaaaatttt tctggatgcc 7680
agcttaaaaa gcgggctcca ttatatttag tggatgaaaa agacctacgg tcgaattttt 7740
cgcccgaggt aatataaatc acctacccag gaataaactg ttcacccaga cacctacgat 7800

gttatatatt ctgtgtggtc cttatttgac aagtgggtct gtggatgcta caatatataa 7860
gacacaaacc cgccccctat tttgggcatg tacgggttac agcagaatta aaaggcttgg 7920
gcgggggata aaacccgtac atgcccaatg tcgtcttaat tttccgtaat tttttgacta 7980
aataaagtta ggaaaatcac tactattaat tatttaatta aaaaactgat ttatttcaat 8040
ccttttagtg atgataatta ataaatsacc gtattctttg aaatggcagt attgataatg 8100
ataaactgag ctccagcttg cataagaaac tttaccgtca taactattac tatttgactc 8160
gaggtcgaab sshttgttcc ctttagtgag ggttaattgc gcgcttggcg taatcatggt 8220
cataacaagg gaaatcactc ccaattaacg cgcgaaccgc attagtacca gtaagctgtt 8280
tcctgtgtga aattgttatc cgctcacaat tccacacaac atatcgacaa aggacacact 8340
ttaacaatag gcgagtgtta aggtgtgttg tatggagccg gaagcataaa gtgtaaagcc 8400
tggggtgcct aatgagtgag gtacctcggc cttcgtattt cacatttcgg accccacgga 8460
ttactcactc catactcaca ttaattgcgt tgcgctcact gcccgctttc cagtcgggaa 8520
acctgagtgt aattaacgca acgcgagtga cgggcgaaag gtcagccctt tggvtgtcgt 8580
gccagctgca ttaatgaatc ggccaacgcg cggggagagg cggtacagca cggtcgacgt 8640
aattacttag ccggttgcgc gcccctctcc gccattgcgt attgggcgct cttccgcttc 8700
ctcgctcact gactcgctgc gctcaacgca taacccgcga gaaggcgaag gagcgagtga 8760
ctgagcgacg cgagggtcgt tcggctgcgg cgagcggtat cagctcactc aaaggcggta 8820
atacccagca agccgacgcc gctcgccata gtcgagtgag tttccgccat tatgggttat 8880
ccacagaatc aggggataac gcaggaaaga acatgtgagc aaaaccaata ggtgtcttag 8940
tcccctattg cgtcctttct tgtacactcg ttttggccag caaaaggcca ggaaccgtaa 9000
aaaggccgcg ttgctggcgt ttttccggtc gttttccggt ccttggcatt tttccggcgc 9060
aacgaccgca aaaaccatag gctccgcccc cctgacgagc atcacaaaaa tcgacgctca 9120
agtcggtatc cgaggcgggg ggactgctcg tagtgttttt agctgcgagt tcagagaggt 9180
ggcgaaaccc gacaggacta taaagatacc aggcgtttcc cccttctcca ccgctttggg 9240
ctgtcctgat atttctatgg tccgcaaagg gggaggaagc tccctcgtgc gctctcctgt 9300
tccgaccctg ccgcttaccg gataccttcg agggagcacg cgagaggaca aggctgggac 9360
ggcgaatggc ctatcctgtc cgcctttctc ccttcgggaa gcgtggcgct ttctcatagc 9420
tcacggacag gcggaaagag ggaagccctt cgcaccgcga aagagtatcg agtggctgta 9480
ggtatctcag ttcggtgtag gtcgttcgct ccaagctggg ctgtcgacat ccatagagtc 9540
aagccacatc cagcaagcga ggttcgaccc gacagtgcac gaaccccccg ttcagcccga 9600
ccgctgcgcc ttatccggta actacacgtg cttggggggc aagtcgggct ggcgacgcgg 9660
aataggccat tgattcgtct tgagtccaac ccggtaagac acgacttatc gccactggca 9720
gcagagcaga actcaggttg ggccattctg tgctgaatag cggtgaccgt cgtcccactg 9780
gtaacaggat tagcagagcg aggtatgtag gcggtgctac agagggtgac cattgtccta 9840
atcgtctcgc tccatacatc cgccacgatg tctcttcttg aagtggtggc ctaactacgg 9900
ctacactaga aggacagtat ttggaagaac ttcaccaccg gattgatgcc gatgtgatct 9960
tcctgtcata aacctatctg cgctctgctg aagccagtta ccttcggaaa aagagttggt 10020
agctatagac gcgagacgac ttcggtcaat ggaagccttt ttctcaacca tcgacttgat 10080
ccggcaaaca aaccaccgct ggtagcggtg gtttttttgt ttgcgaacta ggccgtttgt 10140
ttggtggcga ccatcgccac caaaaaaaca aacgaagcag cagattacgc gcagaaaaaa 10200
aggatctcaa gaagatcctt tgatttcgtc gtctaatgcg cgtctttttt tcctagagtt 10260
cttctaggaa actacttttc tacggggtct gacgctcagt ggaacgaaaa ctcacgttaa 10320
gggagaaaag atgccccaga ctgcgagtca ccttgctttt gagtgcaatt ccctttttgg 10380
tcatgagatt atcaaaaagg atcttcacct agatcctttt aaataaaacc agtactctaa 10440
tagtttttcc tagaagtgga tctaggaaaa tttataaaaa tgaagtttta aatcaatcta 10500
aagtatatat gagtaaactt ggtcattttt acttcaaaat ttagttagat ttcatatata 10560
ctcatttgaa ccagtgacag ttaccaatgc ttaatcagtg aggcacctat ctcagcgatc 10620
tgtcactgtc aatggttacg aattagtcac tccgtggata gagtcgctag acagtatttc 10680

gttcatccat agttgcctga ctccccgtcg tgtagataac tacgataaag caagtaggta 10740
tcaacggact gaggggcagc acatctattg atgcatacgg gagggcttac catctggccc 10800
cagtgctgca atgataccgc gagatatgcc ctcccgaatg gtagaccggg gtcacgacgt 10860
tactatggcg ctctcccacg ctcaccggct ccagatttat cagcaataaa ccagccagcc 10920
ggaagggtgc gagtggccga ggtctaaata gtcgttattt ggtcggtcgg ccttgggccg 10980
agcgcagaag tggtcctgca actttatccg cctccatcca gtctcccggc tcgcgtcttc 11040
accaggacgt tgaaataggc ggaggtaggt cagaattaat tgttgccggg aagctagagt 11100
aagtagttcg ccagttaata gttttaatta acaacggccc ttcgatctca ttcatcaagc 11160
ggtcaattat caaagcgcaa cgttgttgcc attgctacag gcatcgtggt gtcacgctcg 11220
tcgtcgcgtt gcaacaacgg taacgatgtc cgtagcacca cagtgcgagc agcattggta 11280
tggcttcatt cagctccggt tcccaacgat caaggcgagt tacaaaccat accgaagtaa 11340
gtcgaggcca agggttgcta gttccgctca atgtvtgatc ccccatgttg tgcaaaaaag 11400
cggttagctc cttcggtcct ccgatactag ggggtacaac acgttttttc gccaatcgag 11460
gaagccagga ggctavcgtt gtcagaagta agttggccgc agtgttatca ctcatggtta 11520
tggcaggcaa cagtcttcat tcaaccggcg tcacaatagt gagtaccaat accgtccact 11580
gcataattct cttactgtca tgccatccgt aagatgcttt tctgtggtga cgtattaaga 11640
gaatgacagt acggtaggca ttctacgaaa agacacactg gtgagtactc aaccaagtca 11700
ttctgagaat agtgtatgcg gcgacctgac cactcatgag ttggttcagt aagactctta 11760
tcacatacgc cgctgggagt tgctcttgcc cggcgtcaat acgggataat accgcgccac 11820
atagcactca acgagaacgg gccgcagtta tgccctatta tggcgcggtg tatcgtgaac 11880
tttaaaagtg ctcatcattg gaaaacgttc ttcggggcga aaactccttg aaattttcac 11940
gagtagtaac cttttgcaag aagccccgct tttgagtcaa ggatcttacc gctgttgaga 12000
tccagttcga tgtaacccac tcgtgcagtt cctagaatgg cgacaactct aggtcaagct 12060
acattgggtg agcacgaccc aactgatctt cagcatcttt tactttcacc agcgtttctg 12120
ggtgagtggg ttgactagaa gtcgtagaaa atgaaagtgg tcgcaaagac ccactccaaa 12180
aacaggaagg caaaatgccg caaaaaaggg aataagggcg acacgggttt ttgtccttcc 12240
gttttacggc gttttttccc ttattcccgc tgtgccaaat gttgaatact catactcttc 12300
ctttttcaat attattgaag catttattta caacttatga gtatgagaag gaaaaagtta 12360
taataacttc gtaaattcag ggttattgtc tcatgagcgg atacatattt gaatgtattt 12420
agaaaaagtc ccaataacag agtactcgcc tatgtataaa cttacataaa tcttttataa 12480
acaaataggg gttccgcgca catttccccg aaaagtgcca cctgggtatt tgtttatccc 12540
caaggcgcgt gtaaaggggc ttttcacggt ggaccctcct tttcatcacg tgctataaaa 12600
ataattataa tttaaatttt ttaataagga aaagtagtgc acgatatttt tattaatatt 12660
aaatttaaaa aattattaaa tatataaatt aaaaatagaa agtaaaaaaa gaaattaaag 12720
aaaaaaattt atatatttaa tttttatctt tcattttttt ctttaatttc tttttttagt 12780
ttttgttttc cgaagatgta aaagactcta gggggatcgc caacaaatca aaaacaaaag 12840
gcttctacat tttctgagat ccccctagcg gttgttatac taccttttat cttgctcttc 12900
ctgctctcag gtattaatgc cgaatttatg atggaaaata gaacgagaag gacgagagtc 12960
cataattacg gcttaagttt catcttgtct gtgtagaaga ccacacacga aaatcctgtg 13020
attttacaaa gtagaacaga cacatcttct ggtgtgtgct tttaggacac taaaatcatt 13080
ttacttatcg ttaatcgaat gtatatctat ttaatctgct tttcttgtaa aatgaatagc 13140
aattagctta catatagata aattagacga aaagaagtct aataaatata tatgtaaagt 13200
acgctttttg ttgaaatttt ttaaaccaga ttatttatat atacatttca tgcgaaaaac 13260
aactttaaaa aatttgcttt gtttattttt ttttcttcat tccgtaactc ttctaccttc 13320
tttattgaaa caaataaaaa aaaagaagta aggcattgag aagatggaag aaataatact 13380
ttctaaaatc caaatacaaa acataaaaat aaataaacac agagtaatga aagattttag 13440
gtttatgttt tgtattttta tttatttgtg tctcataatt cccaaattat tccatcatta 13500
aaagatacga ggcgcgtgta agttacttaa gggtttaata aggtagtaat tttctatgct 13560

ccgcgcacat tcaatgaggc aagcgatccg tcctaagaaa ccattattat catgacatta 13620
acctattccg ttcgctaggc aggattcttt ggtaataata gtactgtaat tggataaaaa 13680
ataggcgtat cacgaggccc tttcgtcttt ttatccgcat agtgctccgg gaaagcag 13738

<210> 4
<211> 12956
<212> DNA
<213> Artificial Sequence

<220>
<223> Description of Artificial Sequence:Recombinantes
      Plasmid

<220>
<221> misc_feature
<222> (1)..(12956)

<400> 4
sabggacgga tcgggagatc tcccgatccc ctatggtgca ctctcagtac aatcctgcct 60
agccctctag agggctaggg gataccacgt gagagtcatg ttagtgctct gatgccgcat 120
agttaagcca gtatctgctc cctgcttgtg tgttacgaga ctacggcgta tcaattcggt 180
catagacgag ggacgaacac acaaggaggt cgctgagtag tgcgcgagca aaatttaagc 240
tacaacaagg caagcctcca gcgactcatc acgcgctcgt tttaaattcg atgttgttcc 300
gttcgcttga ccgacaattg catgaagaat ctgcttaggg ttaggcgttt tgcgcgaact 360
ggctgttaac gtacttctta gacgaatccc aatccgcaaa acgcsctgct tcgcgatgta 420
cgggccagat atacgcgttg acattgatta ttgacgacga agcgctacat gcccggtcta 480
tatgcgcaac tgtaactaat aactgstagt tattaatagt aatcaattac ggggtcatta 540
gttcatagcc catataatca ataattatca ttagttaatg ccccagtaat caagtatcgg 600
gtatattgga gttccgcgtt acataactta cggtaaatgg cccgcctggc tgaccgacct 660
caaggcgcaa tgtattgaat gccatttacc gggcggaccg actggcccca acgacccccg 720
cccattgacg tcaataatga cgtatgttcc catagtgggt tgctgggggc gggtaactgc 780
agttattact gcatacaagg gtatcaaacg ccaataggga ctttccattg acgtcaatgg 840
gtggagtatt tacggtttgc ggttatccct gaaaggtaac tgcagttacc cacctcataa 900
atgccaaaac tgcccacttg gcagtacatc aagtgtatca tatgccaagt acgccctttg 960
acgggtgaac cgtcatgtag ttcacatagt atacggttca tgcgggccta ttgacgtcaa 1020
tgacggtaaa tggcccgcct ggcattatgc ccagtaggat aactgcagtt actgccattt 1080
accgggcgga ccgtaatacg ggtcatcatg accttatggg actttcctac ttggcagtac 1140
atctacgtat tagtcagtac tggaataccc tgaaaggatg aaccgtcatg tagatgcata 1200
atcagtnctc gctattacca tggtgatgcg gttttggcag tacatcaatg ggcgtggaag 1260
cgataatggt accactacgc caaaaccgtc atgtagttac ccgcacctta gcggtttgac 1320
tcacggggat ttccaagtct ccaccccatt gacgtcaaat cgccaaactg agtgccccta 1380
aaggttcaga ggtggggtaa ctgcagtttg ggagtttgtt ttggcaccaa aatcaacggg 1440
actttccaaa atgtcgtaac cctcaaacaa aaccgtggtt ttagttgccc tgaaaggttt 1500
tacagcatac aactccgccc cattgacgca aatgggcggt aggcgtgtac ggtgggagtg 1560
ttgaggcggg gtaactgcgt ttacccgcca tccgcacatg ccaccctcsa cgtctatata 1620
agcagagctc tctggctaac tagagaaccc actgcttact gcagatatat tcgtctcgag 1680
agaccgattg atctcttggg tgacgaatga cnhgcttatc gaaattaata cgactcacta 1740

tagggagacc caagctggct agccgaatag ctttaattat gctgagtgat atccctctgg 1800
gttcgaccga tcghndmcag tttaaactta agcttatcga taaaatgggc agcttgtact 1860
cggagtaccc aaatttgaat tcgaatagct attttacccg tcgaacatga gcctcatggt 1920
gaaccccaac aaggtccagg aacactataa ttataccaag gagacgctga cttggggttg 1980
ttccaggtcc ttgtgatatt aatatggttc ctctgcgacg aaacgcagga gacgacctcc 2040
cgccaggtgg cctcggcctt catcgtcatc tttgcgtcct ctgctggagg gcggtccacc 2100
ggagccggaa gtagcagtac ctctgttgcg ccattgtggt ggaaaacctt ctggtgctca 2160
ttgcggtggg gagacaacgc ggtaacacca ccttttggaa gaccacgagt aacgccaccc 2220
ccgaaacagc aagttccact cggcaatgta cctgtttctg ggcaacctgg ggctttgtcg 2280
ttcaaggtga gccgttacat ggacaaagac ccgttggacg ccgcctccga tctactggca 2340
ggcgtggcct tcgtagccaa taccttgctc ggcggaggct agatgaccgt ccgcaccgga 2400
agcatcggtt atggaacgam asmactctgg ctctgtcacg ctgaggctga cgcctgtgca 2460
gtggtttgcc cggggagacc gagacagtgc gactccgact gcggacacgt caccaaacgg 2520
gcccmscagg gctctgcctt catcacgctc tcggcctctg tcttcagcct cctggcctcc 2580
cgagacggaa gtagtgcgag agccggagac agaagtcgga ggaccggmsc mscatcgcca 2640
ttgagcgcca cgtggccatt gccaaggtca agctgtatgg cagtagcggt aactcgcggt 2700
gcaccggtaa cggttccagt tcgacatacc gtccgacaag agctgccgca tgcttctgct 2760
catcggggcc tcgtggctca tctgctgttc tcgacggcgt acgaagacga gtagccccgg 2820
agcaccgagt agacgctggt cctcggtggc ctgcccatcc ttggctggaa ctgcctgggc 2880
cacgcgacca ggagccaccg gacgggtagg aaccgacctt gacggacccg gtghctcgag 2940
gcctgctcca ctgtcctgcc tctctacgcc aagcattatg tgctgagctc cggacgaggt 3000
gacaggacgg agagatgcgg ttcgtaatac acgamscgtg cgtggtgacc atcttctcca 3060
tcatcctgtt ggccatcgtg gccctgtcac gcaccactgg tagaagaggt agtaggacaa 3120
ccggtagcac cgggacaacg tgcgcatcta ctgcgtggtc cgctcaagcc acgctgacat 3180
ggccgcctgc acgcgtagat gacgcaccag gcgagttcgg tgcgactgta ccggcggnhc 3240
cgcagacgct agccctgctc aagacggtca ccatcgtgct aggcgtcttg gcgtctgcga 3300
tcgggacgag ttctgccagt ggtagcacga tccgcagaat atcgtctgct ggctgcccgc 3360
cttcagcatc ctccttctgg actatgccta tagcagacga ccgacgggcg gaagtcgtag 3420
gaggaagacc tgatacggag tcccgtccac tcctgcccga tcctctacaa agcccactac 3480
tttttcgccc agggcaggtg aggacgggct aggagatgtt tcgggtgatg aaaaagcggc 3540
rgtctccacc ctgaattccc tgctcaaccc cgtcatctac acgtggcgca gcagaggtgg 3600
gacttaaggg acgagttggg gcagtagatg tgcaccgcgt cstccgggac ctgcggcggg 3660
aggtgcttcg gccgctgcag tgctggcggc cggggccctg gacgccgccc tccacgaagc 3720
cggcgacgtc acgaccgccg gccmasmagg gtgggggtgc aaggacggag gcggggcggg 3780
accccgggcc accacctccc cacccccacg ttcctgcctc cgccccgccc tggggcccgg 3840
tggtggagct gccactccgc agctccagct ccctggagag gggcatgcac atgcccacga 3900
cggtgaggcg tcgaggtcga gggacctctc cccgtacgtg tacgggtgba gtcacccacg 3960
tttctggagg gcaacacggt gttctgagtc gagtctagag cagtgggtgc aaagacctcc 4020
cgttgtgcca caagactcag ctcagatctc mbcggcccgt ttaaacccgc tgatcagcct 4080
cgactgtgcc ttctagttgc cagccgggca aatttgggcg actagtcgga gctgacacgg 4140
aagatcaacg gtcccatctg ttgtttgccc ctcccccgtg ccttccttga ccctggaagg 4200
tgcggtagac aacaaacggg gagggggcac ggaaggaact gggaccttcc acgcactccc 4260
actgtccttt cctaataaaa tgaggaaatt gcatcgcatt gtcgtgaggg tgacaggaaa 4320
ggattatttt actcctttaa cgtagcgtaa cagtgagtag gtgtcattct attctggggg 4380
gtggggtggg gcaggacagc aagactcatc cacagtaaga taagaccccc caccccaccc 4440
cgtcctgtcg ttcggggagg attgggaaga caatagcagg catgctgggg atgcggtggg 4500
ctccccctcc taacccttct gttatcgtcc gtacgacccc tacgccaccc gagvtatggc 4560
ttctgaggcg gaaagaacca gctggggctc tagggggtat ccccataccg aagactccgc 4620

ctttcttggt cgaccccgag atcccccata ggggacgcgc cctgtagcgg cgcattaagc 4680
gcggcgggtg tggtggttac gcgctgcgcg ggacatcgcc gcgtaattcg cgccgcccac 4740
accaccaatg cgcgagcgtg accgctacac ttgccagcgc cctagcgccc gctcctttcg 4800
cttttcgcac tggcgatgtg aacggtcgcg ggatcgcggg cgaggaaagc gaaacttccc 4860
ttcctttctc gccacgttcg ccggctttcc ccgtcaagct ctaagaaggg aaggaaagag 4920
cggtgcaagc ggccgaaagg ggcagttcga gattatcggg ggctcccttt agggttccga 4980
tttagtgctt tacggcacct cgactagccc ccgagggaaa tcccaaggct aaatcacgaa 5040
atgccgtgga gctgcccaaa aaacttgatt agggtgatgg ttcacgtagt gggccatcgc 5100
cctggggttt tttgaactaa tcccactacc aagtgcatca cccggtagcg ggacatagac 5160
ggtttttcgc cctttgacgt tggagtccac gttctttaat agtgtatctg ccaaaaagcg 5220
ggaaactgca acctcaggtg caagaaatta tcacgactct tgttccaaac tggaacaaca 5280
ctcaacccta tctcggtcta ttctctgaga acaaggtttg accttgttgt gagttgggat 5340
agagccagat aagatttgat ttataaggga ttttgccgat ttcggcctat tggttaaaaa 5400
atgaaaacta aatattccct aaaacggcta aagccggata accaattttt tactgctgat 5460
ttaacaaaaa tttaacgcga attaattctg tggaatgtgt gtcacgacta aattgttttt 5520
aaattgcgct taattaagac accttacaca cagtgttagg gtgtggaaag tccccaggct 5580
ccccagcagg cagaagtatg caaacaatcc cacacctttc aggggtccga ggggtcgtcc 5640
gtcttcatac gtttgcatgc atctcaatta gtcagcaacc aggtgtggaa agtccccagg 5700
ctcccgtacg tagagttaat cagtcgttgg tccacacctt tcaggggtcc gaggccagca 5760
ggcagaagta tgcaaagcat gcatctcaat tagtcagcaa ccatggtcgt ccgtcttcat 5820
acgtttcgta cgtagagtta atcagtcgtt ggtaagtccc gcccctaact ccgcccatcc 5880
cgcccctaac tccgcccagt tccgtcaggg cggggattga ggcgggtagg gcggggattg 5940
aggcgggtca aggcncccca ttctccgccc catggctgac taattttttt tatttatgca 6000
gaggccgggt aagaggcggg gtaccgactg attaaaaaaa ataaatacgt ctccgggagg 6060
ccgcctctgc ctctgagcta ttccagaagt agtgaggagg ctttttctcc ggcggagacg 6120
gagactcgat aaggtcttca tcactcctcc gaaaaamasm atggaggcct aggcttttgc 6180
aaaaagctcc cgggagcttg tatatccatt tacctccgga tccgaaaacg tttttcgagg 6240
gccctcgaac atataggtaa abctcggatc tgatcaagag acaggatgag gatcgtttcg 6300
catgattgaa caaagcctag actagttctc tgtcctactc ctagcaaagc gtactaactt 6360
gttgatggat tgcacgcagg ttctccggcc gcttgggtgg agaggctatt cggctaccta 6420
acgtgcgtcc aagaggccgg cgaacccacc tctccgataa gccctatgac tgggcacaac 6480
agacaatcgg ctgctctgat gccgccgtgt tccgatactg acccgtgttg tctgttagcc 6540
gacgagacta cggcggcaca aggggctgtc agcgcagggg cgcccggttc tttttgtcaa 6600
gaccgacctg tccccgacag tcgcgtcccc gcgggccaag aaaaacagtt ctggctggac 6660
aggstmscgg tgccctgaat gaactgcagg acgaggcagc gcggctatcg tggctggccc 6720
acgggactta cttgacgtcc tgctccgtcg cgccgatagc accgaccgms cvcacgacgg 6780
gcgttccttg cgcagctgtg ctcgacgttg tcactgaagc gggtgctgcc cgcaaggaac 6840
gcgtcgacac gagctgcaac agtgacttcg ccgaagggac tggctgctat tgggcgaagt 6900
gccggggcag gatctcctgt cacttccctg accgacgata acccgcttca cggccccgtc 6960
ctagaggaca gttctcacct tgctcctgcc gagaaagtat ccatcatggc tgatgcaatg 7020
cgagagtgga acgaggacgg ctctttcata ggtagtaccg actacgttac gcgcggctgc 7080
atacgcttga tccggctacc tgcccattcg accaccaagc gacgccgacg tatgcgaact 7140
aggccgatgg acgggtaagc tggtggttcg ctaacatcgc atcgagcgag cacgtactcg 7200
gatggaagcc ggtcttgtcg atttgtagcg tagctcgctc gtgcatgagc ctaccttcgg 7260
ccagaacagc tacaggatga tctggacgaa gagcatcagg ggctcgcgcc agccgaactg 7320
ttgtcctact agacctgctt ctcgtagtcc ccgagcgcgg tcggcttgac aabsshnccg 7380
ccaggctcaa ggcgcgcatg cccgacggcg aggatctcgt cgtgacccgc ggtccgagtt 7440
ccgcgcgtac gggctgccgc tcctagagca gcactgggnc atggcgatgc ctgcttgccg 7500

aatatcatgg tggaaaatgg ccgcttttct taccgctacg gacgaacggc ttatagtacc 7560
accttttacc ggcgaaaaga ggattcatcg actgtggccg gctgggtgtg gcggaccgct 7620
atcaggacat cctaagtagc tgacaccggc cgacccacac cgcctggcga tagtcctgta 7680
agcgttggct acccgtgata ttgctgaaga gcttggcggc gaatgggctg tcgcaaccga 7740
tgggcactat aacgacttct cgaaccgccg cttacccgac accgcttcct cgtgctttac 7800
ggtatcgccg ctcccgattc gcagcgcatc tggcgaagga gcacgaaatg ccatagcggc 7860
gagggctaag cgtcgcgtag bstbgccttc tatcgccttc ttgacgagtt cttctgagcg 7920
ggactctggg gttccggaag atagcggaag aactgctcaa gaagactcgc cctgagaccc 7980
caagbstbga aatgaccgac caagcgacgc ccaacctgcc atcacgagat ttcgattcct 8040
ttactggctg gttcgctgcg ggttggacgg tagtgctcta aagctaagca ccgccgcctt 8100
ctatgaaagg ttgggcttcg gaatcgtttt ccgggacggt ggcggcggaa gatactttcc 8160
aacccgaagc cttagcaaaa ggccctgccc ggctggatga tcctccagcg cggggatctc 8220
atgctggagt tcttcgccgg ccgacctact aggaggtcgc gcccctagag tacgacctca 8280
agaagcggca ccccaacttg tttattgcag cttataatgg ttacaaataa agcaataggt 8340
ggggttgaac aaataacgtc gaatattacc aatgtttatt tcgttatcca tcacaaattt 8400
cacaaataaa gcattttttt cactgcattc tagttgtggt agtgtttaaa gtgtttattt 8460
cgtaaaaaaa gtgacgtaag atcaacacsa gtttgtccaa actcatcaat gtatcttatc 8520
atgtctgtat accgtcgacc caaacaggtt tgagtagtta catagaatag tacagacata 8580
tggcagctgg tctagctaga gcttggcgta atcatggtca tagctgtttc ctgtgtgaaa 8640
agatcgatct cgaaccgcat tagtaccagt atcgacaaag gacacacttt ttgttatccg 8700
ctcacaattc cacacaacat acgagccgga agcataaagt aacaataggc gagtgttaag 8760
gtgtgttgta tgctcggcct tcgtatttca gtaaagcctg gggtgcctaa tgagtgagct 8820
aactcacatt aattgcgttg catttcggac cccacggatt actcactcga ttgagtgtaa 8880
ttaacgcaac vcgctcactg cccgctttcc agtcgggaaa cctgtcgtgc cagctgcatt 8940
agcgagtgac gggcgaaagg tcagcccttt ggacagcacg gtcgacgtaa tatgaatcgg 9000
ccaacgcgcg gggagaggcg gtttgcgtat tgggcgctct ttacttagcc ggttgcgcgc 9060
ccctctccgc caaacgcata acccgcgaga accgcttcct cgctcactga ctcgctgcgc 9120
tcggtcgttc ggctgcggcg aggcgaagga gcgagtgact gagcgacgcg agccagcaag 9180
ccgacgccgc tgcggtatca gctcactcaa aggcggtaat acggttatcc acagaatcag 9240
gcgccatagt cgagtgagtt tccgccatta tgccaatagg tgtcttagtc cggataacgc 9300
aggaaagaac atgtgagcaa aaggccagca aaaggccagg acctattgcg tcctttcttg 9360
tacactcgtt ttccggtcgt tttccggtcc taccgtaaaa aggccgcgtt gctggcgttt 9420
ttccataggc tccgcccccc ttggcatttt tccggcgcaa cgaccgcaaa aaggtatccg 9480
aggcgggggg agacgagcat cacaaaaatc gacgctcaag tcagaggtgg cgaaacccga 9540
cctgctcgta gtgtttttag ctgcgagttc agtctccacc gctttgggct gaggactata 9600
aagataccag gcgtttcccc ctggaagctc cctcgtgcgc ttcctgatat ttctatggtc 9660
cgcaaagggg gaccttcgag ggagcacgcg actcctgttc cgaccctgcc gcttaccgga 9720
tacctgtccg cctttctccc tgaggacaag gctgggacgg cgaatggcct atggacaggc 9780
ggaaagaggg atcgggaagc gtggcgcttt ctcatagctc acgctgtagg tatctcagtt 9840
cagcccttcg caccgcgaaa gagtatcgag tgcgacatcc atagagtcaa gggtgtaggt 9900
cgttcgctcc aagctgggct gtgtgcacga accccccgtt cccacatcca gcaagcgagg 9960
ttcgacccga cacacgtgct tggggggcaa gagcccgacc gctgcgcctt atccggtaac 10020
tatcgtcttg agtccaaccc gtcgggctgg cgacgcggaa taggccattg atagcagaac 10080
tcaggttggg cgtaagacac gacttatcgc cactggcagc agccactggt aacaggatta 10140
gcattctgtg ctgaatagcg gtgaccgtcg tcggtgacca ttgtcctaat ccagagcgag 10200
gtatgtaggc ggtgctacag agttcttgaa gtggtggcct agtctcgctc catacatccg 10260
ccacgatgtc tcaagaactt caccaccgga tactacggct acactagaag aacagtattt 10320
ggtatctgcg ctctgctgaa gtgatgccga tgtgatcttc ttgtcataaa ccatagacgc 10380

gagacgactt cccagttacc ttcggaaaaa gagttggtag ctcttgatcc ggcaaacaaa 10440
cggtcaatgg aagccttttt ctcaaccatc gagaactagg ccgtttgttt gcaccgctgg 10500
tagcggtttt tttgtttgca agcagcagat tacgcgcaga agtggcgacc atcgccaaaa 10560
aaacaaacgt tcgtcgtcta atgcgcgtct taaaaaggat ctcaagaaga tcctttgatc 10620
ttttctacgg ggtctgacgc ttttttccta gagttcttct aggaaactag aaaagatgcc 10680
ccagactgcg acagtggaac gaaaactcac gttaagggat tttggtcatg agattatcaa 10740
agtcaccttg cttttgagtg caattcccta aaaccagtac tctaatagtt taaggatctt 10800
cacctagatc cttttaaatt aaaaatgaag ttttaaatca attcctagaa gtggatctag 10860
gaaaatttaa tttttacttc aaaatttagt ttctaaagta tatatgagta aacttggtct 10920
gacagttacc aatgcttaat cagatttcat atatactcat ttgaaccaga ctgtcaatgg 10980
ttacgaatta gagtgaggca cctatctcag cgatctgtct atttcgttca tccatagttg 11040
ctcactccgt ggatagagtc gctagacaga taaagcaagt aggtatcaac gctgactccc 11100
cgtcgtgtag ataactacga tacgggaggg cttaccatct ggactgaggg gcagcacatc 11160
tattgatgct atgccctccc gaatggtaga cgccccagtg ctgcaatgat accgcgagac 11220
ccacgctcac cggctccaga tcggggtcac gacgttacta tggcgctctg ggtgcgagtg 11280
gccgaggtct attatcagca ataaaccagc cagccggaag ggccgagcgc agaagtggtc 11340
caatagtcgt tatttggtcg gtcggccttc ccggctcgcg tcttcaccag gtgcaacttt 11400
atccgcctcc atccagtcta ttaattgttg ccgggaagct aacgttgaaa taggcggagg 11460
taggtcagat aattaacaac ggcccttcga tgagtaagta gttcgccagt taatagtttg 11520
cgcaacgttg ttgccattgc tctcattcat caagcggtca attatcaaac gcgttgcaac 11580
aacggtaacg aacaggcatc gtggtgtcac gctcgtcgtt tggtatggct tcattcagct 11640
ctgtccgtag caccacagtg cgagcagcaa accataccga agtaagtcga gcggttccca 11700
acgatcaagg cgagttacat gatcccccat gttgtgcaaa agccaagggt tgctagttcc 11760
gctcaatgta ctagggggta caacacgttt tvaagcggtt agctccttcg gtcctccgat 11820
cgttgtcaga agtaagttgg ccttcgccaa tcgaggaagc caggaggcta gcaacagtct 11880
tcattcaacc gggcagtgtt atcactcatg gttatggcag cactgcataa ttctcttact 11940
gtcgtcacaa tagtgagtac caataccgtc gtgacgtatt aagagaatga cacatgccat 12000
ccgtaagatg cttttctgtg actggtgagt actcaaccaa gtgtacggta ggcattctac 12060
gaaaagacac tgaccactca tgagttggtt cacattctga gaatagtgta tgcggcgacc 12120
gagttgctct tgcccggcgt cagtaagact cttatcacat acgccgctgg ctcaacgaga 12180
acgggccgca gtatacggga taataccgcg ccacatagca gaactttaaa agtgctcatc 12240
attatgccct attatggcgc ggtgtatcgt cttgaaattt tcacgagtag tatggaaaac 12300
gttcttcggg gcgaaaactc tcaaggatct taccgctgtt gaaccttttg caagaagccc 12360
cgcttttgag agttcctaga atggcgacaa ctgatccagt tcgatgtaac ccactcgtgc 12420
acccaactga tcttcagcat ctctaggtca agctacattg ggtgagcacg tgggttgact 12480
agaagtcgta gatttacttt caccagcgtt tctgggtgag caaaaacagg aaggcaaaat 12540
gcaaatgaaa gtggtcgcaa agacccactc gtttttgtcc ttccgtttta cgcgcaaaaa 12600
agggaataag ggcgacacgg aaatgttgaa tactcatact ctgcgttttt tcccttattc 12660
ccgctgtgcc tttacaactt atgagtatga gatccttttt caatattatt gaagcattta 12720
tcagggttat tgtctcatga gcaggaaaaa gttataataa cttcgtaaat agtcccaata 12780
acagagtact cgggatacat atttgaatgt atttagaaaa ataaacaaat aggggttccg 12840
cgcctatgta taaacttaca taaatctttt tatttgttta tccccaaggc gcsacacatt 12900
tccccgaaaa gtgccacctg acgtcgtgta aaggggcttt tcacggtgga ctgcag 12956